# EUROPÄISCHE PATENTSCHRIFT

(11) **EP 3 606 450 B1**
(45) Veröffentlichungstag und Bekanntmachung des Hinweises auf die Patenterteilung: **01.12.2021**
(21) Anmeldenummer: 18708077.5
(22) Anmeldetag: 22.02.2018
(51) Int. Cl.: A61B 17/322

(54) **VORRICHTUNG ZUR ERZEUGUNG EINER MIKROGRAFTMATRIX AUS VOLLHAUT**
DEVICE FOR CREATING A MICROGRAFT MATRIX FROM FULL-THICKNESS SKIN
PROCÉDÉ POUR LA PRODUCTION D'UNE MATRICE DE MICROGREFFE À PARTIR D'UNE PEAU COMPLÈTE

(30) Priorität: 23.03.2017 DE 102017106310
(43) Veröffentlichungstag der Anmeldung: 12.02.2020
(73) Patentinhaber: Lavenir Bioscience AG, 22417 Hamburg (DE)
(72) Erfinder: DEPFENHART, Markus, 22417 Hamburg (DE); MÜLLER, Jörg, 21224 Buchholz (DE); TRIEU, Hoc-Kiem, 21394 Westergellersen (DE); SPANIC, Matthias, 80337 München (DE)
(74) Vertreter: Heinemeyer, Karsten
(86) Internationale Anmeldenummer: PCT/EP2018/054404
(87) Internationale Veröffentlichungsnummer: WO 2018/172015

(56) Entgegenhaltungen:
- WO-A1-2016/033584
- US-A- 4 270 540
- US-A1- 2014 081 251

## Beschreibung

Die vorliegende Erfindung betrifft ein Vorrichtungsset zur Erzeugung einer Mikrograftmatrix aus Vollhaut umfassend ein Folienset mit einer ersten und einer zweiten Folie, die koplanar sind, aneinanderhaften und voneinander abziehbar sind, eine Schneidevorrichtung, mit der eine Vielzahl hohlzylindrischer Schnitte durch das Folienset hindurch, die auf der Haut klebt, in die Haut hinein erzeugt werden, und eine dritte Folie, die mit der zweiten Folie eine Klebeverbindung eingeht.

Als Stand der Technik sind verschiedene Vorrichtungen und Verfahren zur Erzeugung einer Mikrograftmatrix oder Transplantationsmatrix aus Vollhaut oder Spalthaut, die eine obere Haut umfasst, bekannt. Spalthautteile, die die Epidermis und die obere papilläre aber nicht die retikuläre Dermis umfassen, sind jedoch bei weitem weniger wirksam für eine Transplantation als Vollhautteile oder Vollhautstanzteile. Zur Erzeugung der Mikrograftmatrix oder Transplantationsmatrix aus gesunder Haut stehen besonders folgende Anforderungen im Vordergrund: geringe Morbidität des Spenderhautareals, geringe Morbidität der entnommenen Hautteile, geringe Belastung des Patienten, möglichst schnelles Verfahren.

US 2014 0277 454 A1 offenbart eine Vorrichtung und ein Verfahren, bei dem eine Platte mit einer Vielzahl an Löchern oder Vertiefungen auf die Haut aufgelegt wird, wobei die Löcher durch Kanäle miteinander verbunden sind und mit einem Unterdruck beaufschlagt sind. Durch den Unterdruck werden eine Vielzahl von Hautteilen in die Löcher oder Vertiefungen herausgezogen und können abgeschnitten oder abgeschält werden. Es wird dabei aber nur Spalthaut entnommen.

G. U. Seip offenbart in Phlebologie 2016; 45: S. 100-105, Schattauer-Verlag (http://dx.doi.org/10.12687/phleb2303-2-2016) mit dem Titel "Plastic chronic wound management with Cellutome" ein Gerät und ein Verfahren ähnlich zu US 2014 0277 454 A1, mit dem in einem ersten Schritt erzeugte Hauterhebungen mit einer Klebefolie überdeckt und dann an der Folie anhaftend in einem zweiten Schritt abgeschnitten, entnommen und transplantiert werden können.

US 2014 0 277 454 A1 offenbart eine Vorrichtung und ein Verfahren, bei dem mit einer Platte mit einer Vielzahl an Löchern eine Vielzahl der Hautteile vom Spenderhautareal entnommen wird, um dann auf ein Empfängerhautareal aufgebracht zu werden. Über der Platte ist ein Gehäuse angeordnet, das eine Rückseite der Platte umgibt und gegen das jeweilige Hautareal abgedichtet, so dass durch in der Platte eingebrachte Kanäle Gewebeflüssigkeit herausgesaugt und verteilt werden kann.

US 2015 0 238 212 A1 offenbart verschiedene Verfahren, mit dem Vorrichtungen zur Erzeugung der Mikrograftmatrix oder Transplantationsmatrix aus Vollhaut oder Spalthaut hergestellt werden können

US 2014 039 523 A1 offenbart eine Vorrichtung und ein Verfahren, bei dem durch einen Nadelroller mit einer Vielzahl an Hohlnadeln bei einem Rollen über die Haut Hautteile aus der Haut ausgestanzt werden. Mit dem Nadelroller können sowohl Spalthaut- als auch Vollhautstanzteile entnommen werden; ein Herausnehmen und Verwerten der Vollhautstanzteile aus den Nadeln ist jedoch extrem mühsam bis unmöglich. Der Nadelroller ist eher für eine Hautdurchlöcherung gedacht als für eine Hautentnahme zwecks Transplantation.

Ähnlich dem Verfahren nach US 2014 039 523 A1 aber apparativ wesentlich aufwendiger sind Mikrostanzen bekannt, mit denen Spalt und Vollhautstanzteile aus einer Haut sequenziell matrixförmig entnommen werden können, die dann aber einzeln zwecks einer Transplantation weiterverarbeitet werden müssen. Ein solches Verfahren ist apparativ als auch zeitlich sehr aufwendig.

DE 10 2009 018 940 A1 offenbart eine Vorrichtung mit einer Hohlstanze, die eine Schneide mit einer gezackten Umrissform aufweist, so dass die Haut entsprechend sternförmig ausgeschnitten wird, um die Umrissform auf der Haut nach einem Ausstanzen zu verwischen. Es können damit auch größere Hautareale gestanzt oder geschnitten und dann abgeschält werden.

WO 2016/033584 A1 offenbart ein Vorrichtungsset zur Erzeugung einer Mikrograftmatrix mit einer Vielzahl an Vollhautstanzteilen aus einer Haut eines Hautentnahmeareals, umfassend: eine Folie, wobei die Folie ausgebildet ist, mit einer flächigen Unterseite auf die Haut geklebt zu werden; ein Klebemittel zum Aufkleben der Unterseite der Folie auf die Haut; und eine Schneidevorrichtung, wobei die Schneidevorrichtung ausgebildet ist, eine Vielzahl an zueinander parallelen hohlzylindrischen Schnitten mit jeweils einem Hohlzylinderinnendurchmesser und einem Hohlzylinderabstand zueinander bis zu einer vorbestimmten Tiefe in der Haut zu erzeugen, so dass die Vielzahl der hohlzylindrischen Schnitte matrixförmig im Hautentnahmeareal erzeugt wird.

Generell ist mit Stanzen im Zusammenhang mit dieser Patentanmeldung immer ein einseitiges Stanzen von oben in die Haut oder ein In-die-Haut-Schneiden von oben senkrecht in die Haut gemeint, um ein dabei erzeugtes Hautteil oder Hautstanzteil dann abheben und bevorzugt transplantieren zu können.

Die bekannten Verfahren lassen nur eine relativ begrenzte Hautentnahme von 2-3% der Körperoberfläche zu, die auch oft zu gering ist für eine Therapie bei größeren Wundflächen wie beispielsweise großflächigen Verbrennungen. Dabei kommt es auch oft zu einer unerwünschten nachträglichen Narbenbildung.

Die Aufgabe der Erfindung, um die Nachteile aus dem Stand der Technik zu beseitigen, besteht daher in der Bereitstellung eines Vorrichtungssets zur Erzeugung einer

Mikrograftmatrix aus Vollhaut, die mit möglichst geringem apparativen Aufwand, möglichst schnell, mit einem möglichst geringem Hebedefekt, und das über ein möglichst großes Hautentnahmeareal erzeugt werden kann.

Die vorstehende Aufgabe wird von einer Vorrichtung gemäß den Merkmalen des unabhängigen Anspruchs 1 gelöst. Weitere vorteilhafte Ausführungsformen der Erfindung sind in den abhängigen Ansprüchen angegeben.

Erfindungsgemäß wird ein Vorrichtungsset zur Erzeugung einer Mikrograftmatrix mit einer Vielzahl an Vollhautstanzteilen aus einer Haut eines Hautentnahmeareals zur Verfügung gestellt, das folgende Komponenten umfasst:
ein Folienset mit einer vorbestimmten Dicke aus mindestens einer ersten Folie und einer zweiten Folie, wobei die erste Folie ausgebildet ist, mit einer flächigen Unterseite auf die Haut geklebt zu werden und mit einer gegenüberliegenden Oberseite koplanar mit einer Unterseite der zweiten Folie mit einer zweiten Klebefestigkeit aneinanderzuhaften und voneinander abziehbar zu sein;
ein Klebemittel zum Aufkleben der Unterseite der ersten Folie auf die Haut, wobei zwischen der Unterseite der ersten Folie und der Haut eine erste Klebefestigkeit erzeugt wird, die größer als die zweite Klebefestigkeit ist, so dass die zweite Folie von der auf der Haut klebenden ersten Folie abziehbar ist;
eine Schneidevorrichtung mit einem Adapter, der ausgebildet ist, durch Anpressen an das Folienset, das auf die Haut geklebt ist, unter Berücksichtigung der Dicke des Foliensets im Hautentnahmeareal einen vordefinierten Abstand zur darunter liegenden Haut herzustellen, wobei die Schneidvorrichtung ausgebildet ist, eine Vielzahl an zueinander parallelen hohlzylindrischen Schnitten mit jeweils einem Hohlzylinderinnendurchmesser und einem Hohlzylinderabstand zueinander senkrecht zum Folienset und bis zu einer vorbestimmten Tiefe in der darunterliegenden Haut zu erzeugen, so dass die Vielzahl der hohlzylindrischen Schnitte matrixförmig im Hautentnahmeareal erzeugt wird, um durch die hohlzylindrischen Schnitte das Folienset in jeweilige innere Abschnitte mit einem jeweiligen inneren Abschnitt der zweiten Folie, der über dem jeweiligen geschnittenen Vollhautstanzteil liegt, und in einen jeweiligen äußeren Abschnitt mit einem äußeren Abschnitt der zweiten Folie zu unterteilen; und
eine dritte Folie mit einer Größe, die zumindest so groß wie das Entnahmeareal ist und die ausgebildet ist, in Kontakt mit einer Oberseite der zweiten Folie, die der Unterseite der zweiten Folie gegenüberliegt, eine dritte Klebefestigkeit zu erzeugen, wobei die erste, die zweite und die dritte Klebefestigkeit größer als eine vierte Haftfestigkeit ist, mit der die Vollhautstanzteile in der Haut gehalten werden.

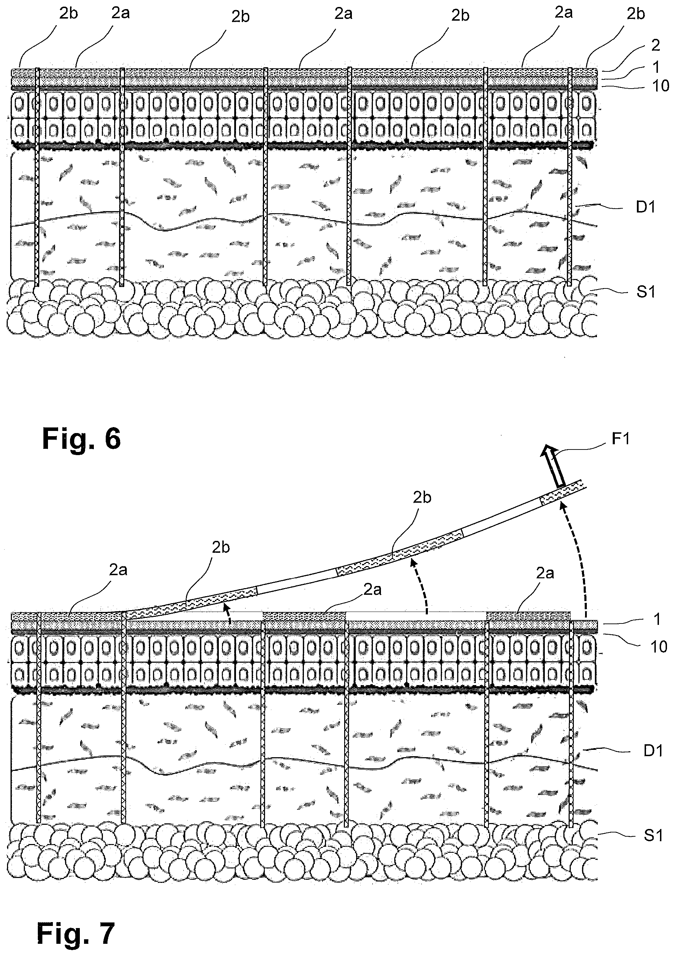

Das erfindungsgemäße Vorrichtungsset ist besonders einfach und kostengünstig und kann schnell angewendet werden, da die Vielzahl der damit erzeugten Vollhautstanzteile in einfacher Weise gemeinsam mit der dritten Folie verbunden und damit gemeinsam entnommen werden können. So kann mit einem Vorgang eine gesamte Matrix aus Vollhautstanzteilen entnommen werden, welche die Mikrograftmatrix darstellt. Das Folienset mit der ersten und zweiten Folie ist einfach und kostengünstig und kann einfach auf die Haut, die eine Spenderhaut ist, aufgeklebt werden. Indem in der Anwendung des Vorrichtungssets mit der Schneidevorrichtung hohlzylindrische Schnitte senkrecht in die vorbestimmte Tiefe der Haut eingebracht werden, wird zuerst auch das Folienset aus der ersten und zweiten Folie entlang der Schnittlinie durchschnitten. Dadurch werden innere Abschnitte und ein äußerer Abschnitt der ersten und der zweiten Folie erzeugt. Wenn die Schnitte in die vorbestimmte Tiefe der Haut vorgenommen werden, werden die Vollhautstanzteile mit einer zylindrischen Form erzeugt. Dabei sind die Vollhautstanzteile zwar mit dem jeweiligen inneren Abschnitt der ersten und der zweiten Folie verbunden, sie sind jedoch nicht mit dem Rest des Foliensets verbunden, so dass sie zusammen mit den jeweiligen inneren Abschnitten der ersten und darüber liegenden zweiten Folie herausgezogen werden können. Indem nun der verbleibende äußere Abschnitt der zweiten Folie, der sich außerhalb der annularen Schnitte in die Haut befindet, von der ersten Folie abgezogen wird, sind die inneren Abschnitte der zweiten Folie in Bezug zur ersten Folie erhöht, mit Leerräumen dazwischen. In Fig. 7 ist das gut erkennbar. Nun kann die dritte Folie einfach auf die erhöhten inneren Abschnitte der zweiten Folie aufgelegt und damit verklebt werden. Bei einem anschließenden Abziehen der dritten Folie von der Haut werden die Vollhautstanzteile als die Mikrograftmatrix herausgezogen. Mit dem erfindungsgemäßen Vorrichtungsset läßt sich also die Vielzahl der Vollhautstanzteile durch die Verklebung mit der dritten Folie in ihrer Lage zueinander und erhaltener axialer Ausrichtung halten und aus der Haut ziehen. Vorteilhafterweise braucht dann auch nicht jedes Hautstanzteil für sich transplantiert zu werden, sondern es kann die Matrix der Vollhautstanzteile oder die "Mikrograftmatrix" insgesamt auf die zu behandelnde Wunde gelegt und damit verklebt werden. Danach können die Hautstanzteile dann in der Wunde anwachsen und dazwischen auswachsen. Das erfindungsgemäße Vorrichtungsset ermöglicht so eine viel kürzere Behandlungszeit und eine viel geringe Morbidität, als es bei Vorrichtungen und Verfahren gemäß dem Stand der Technik möglich ist.

Die mit der Erfindung erzielten Vorteile bestehen insbesondere darin, dass durch einfache und kostengünstige Mittel in der Kombination eine besonders gute, schnelle und großflächige Vollhautentnahme ermöglicht wird.

Das erfindungsgemäße Vorrichtungsset ermögiicht auch durch die Vielzahl der Vollhautstanzteile bei geeigneten Abständen zu einander, dass sie über große Hautentnahmeareale entnommen werden können. Dadurch lässt sich ein Gesamtspenderareal von 2 - 3% der Körperoberfläche, wie im Stand der Technik, nunmehr auf 20 - 50% erweitern, wodurch die Morbidität von Patienten drastisch reduziert wird.

Ebenso wird eine Narbenbildung vollständig vermieden, da die Haut im Spenderareal nicht großflächig in Kontinuitatem sondern nur mikrofraktioniert und partiell durch die Vielzahl voneinander beabstandeter Kavitäten, die durch die Entnahme der Vollhautstanzteile entstehen, entnommen wird. Es ist bekannt, dass vollschichtige Hautdefekte, die einen Durchmesser kleiner oder gleich 300 Mikrometer aufweisen, grundsätzlich narbenfrei abheilen. So kann die Haut nach der Defekterzeugung mit dem erfindungsgemäßen Vorrichtungsset natürlich verjüngen und narbenfreie (Restitutio ad integrum) nachwachsen. Auch im Wundbereich, dort, wo die Vollhautstanzteile hin transplantiert werden, kann die Haut zwischen den Vollhautstanzteilen gut auswachsen.

Vorteilhaft ist auch, dass Hautstanzteile beispielsweise mit Haaren und sonstigen retikulären dermalen Hautbestandteilen entnommen und verpflanzt werden können und auf diese Weise auch eine Haartransplantation durchgeführt werden kann.

Bevorzugt ist die Schneidevorrichtung eine Trägerplatte mit einer Vielzahl an Mikrohohlstanzen, die matrixförmig angeordnet sind. So lassen sich viele Schnitte auf einmal und kostengünstig vornehmen.

Alternativ bevorzugt wird als der Schneidevorrichtung ein Laser mit einer Optik verwendet, die die Vielzahl der Schnitte in die vorbestimmte Tiefe vornehmen.

Weitere Vorteile sind in der detaillierten Beschreibung zu entnehmen.

Bevorzugte Ausführungsformen gemäß der vorliegenden Erfindung sind in nachfolgenden Zeichnungen und in einer detaillierten Beschreibung dargestellt, sie sollen aber die vorliegende Erfindung nicht ausschließlich darauf begrenzen.

Es zeigen
- Fig. 1: eine perspektivische Ansicht eines Schnittbilds einer menschlichen Haut mit Epidermis, retikulärer Dermis und Subcutis;
- Fig. 2: ein Schnittbild der menschlichen Haut mit Epidermis, papillären und retikulären Dermis und Subcutis, wobei auf der Hautoberfläche schichtweise eine Klebeschicht, eine erste und eine zweite Folie aufgebracht ist, wobei die erste und die zweite Folie zusammen ein Folienset darstellen;
- Fig. 3: eine Seitenansicht des Schnittbilds aus Fig. 2 mit einer darüber angeordneten Schneidevorrichtung, die eine Trägerplatte mit einer Vielzahl an Mikrohohlstanzen umfasst, die mit einem Abschnitt mit einer Schneidekante zum Folienset und der Haut hin ausgerichtet sind und mit dem gegenüberliegenden Abschnitt über Aktuatoren mit der Trägerplatte verbunden sind, wobei die Trägerplatte über einen Verschiebeaktuator von einem zweiten Träger gehalten wird;
- Fig. 4: eine Seitenansicht des Schnittbilds aus Fig. 3, wobei die Trägerplatte die Mikrohohlstanzen in die Haut bis zu einer vorbestimmten Tiefe, die bevorzugt am Ende der Dermis liegt, eingefahren ist und dadurch Vollhautstanzteile erzeugt;
- Fig. 5: eine Seitenansicht des Schnittbilds aus Fig. 4, wobei die Trägerplatte die Mikrohohlstanzen nach dem Einfahren in die Haut wieder herausgezogen und gleichzeitig einen äußeren Abschnitt der zweiten Folie mit von der ersten Folie abgezogen hat, wobei die Vollhautstanzteile nur noch unten und seitlich durch Reibung in der Haut haften;
- Fig. 6: ein seitliches Schnittbild der Haut mit dem Folienset darauf nach Fig. 4, wobei die Mikrohohlstanzen herausgezogen sind und das Folienset mit der ersten und zweiten Folie durch jeweilige Schnitte unterteilt ist in jeweils innere Abschnitte und jeweils einen äußeren, restlichen Abschnitt der jeweiligen ersten und zweiten Folie;
- Fig. 7: ein seitliches Schnittbild der Haut mit dem Folienset aus Fig. 6, wobei der äußere Abschnitt der zweiten Folie vom Folienset teilweise abgezogen ist, wobei die inneren Abschnitte der zweiten Folie auf der ersten Folie verbleiben;
- Fig. 8: ein seitliches Schnittbild der Haut mit dem Folienset aus Fig. 7, wobei der äußere Abschnitt der zweiten Folie von der ersten Folie abgezogen ist und eine dritte Folie auf die inneren Abschnitte der zweiten Folie aufgelegt ist und mit Licht einer Lichtquelle bestrahlt wird;
- Fig. 9: ein seitliches Schnittbild der Haut mit der ersten, zweiten und dritten Folie und der Lichtquelle aus Fig. 8, wobei zwischen der zweiten Folie und der dritten Folie eine zweite Klebeschicht eingebracht ist;
- Fig. 10: ein seitliches Schnittbild der Haut mit der ersten, zweiten und dritten Folie aus Fig. 8 nach der Bestrahlung;
- Fig. 11: ein seitliches Schnittbild der Haut mit der ersten, zweiten und dritten Folie aus Fig. 10, wobei die dritte Folie mit den daran anhaftenden Vollhautstanzteilen teilweise herausgezogen ist;
- Fig. 12: ein seitliches Schnittbild der dritten Folie aus Fig. 11, wobei die dritte Folie mit den daran anhaftenden Vollhautstanzteilen herausgezogen ist und eine Mikrograftmatrix darstellt;
- Fig. 13: eine Ansicht von oben als Ausschnitt auf ein Spenderhautareal, aus der die Vollhautstanzteile herausgezogen sind, wobei zurückbleibende Kavitäten zu sehen sind, und auf der noch die erste Folie klebt;
- Fig. 14: eine Seitenansicht der Schneidevorrichtung aus Fig. 3, wobei die Mikrohohlstanzen an ihrem oberen Ende jeweils eine Kontaktstelle aufweisen, die mit einer jeweiligen Gegenkontaktstelle an dem jeweiligen Aktuator verbunden und davon wieder gelöst werden können;
- Fig. 15: eine Seitenansicht eines Teils einer bevorzugten Schneidevorrichtung ähnlich zu der aus Fig. 3, wobei die Mikrohohlstanzen aus einem Blech mit einer Abrisskante tiefgezogen oder ausgestanzt sind und eine Einheit bilden, die mit der Trägerplatte verbunden werden kann;
- Fig. 16: eine Seitenansicht eines Teils einer anderen bevorzugten Schneidevorrichtung ähnlich zu der aus Fig. 3 und 15, wobei in der jeweiligen Mikrohohlstanze jeweils ein Mandrin angeordnet ist, der in Längsrichtung der Mikrohohlstanze beweglich verfahren werden kann; zudem sind die Schneiden an einem unteren Ende nach oben außen zunehmend.

### Detaillierte Beschreibung von Ausführunasbeispielen

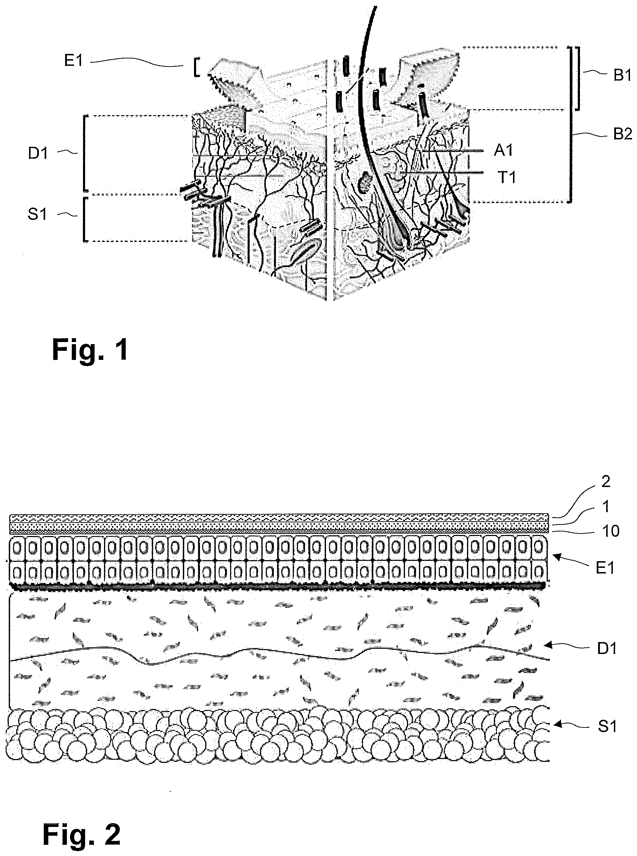

Fig. 1 zeigt eine perspektivische Ansicht eines Schnittbilds einer menschlichen Haut mit Epidermis E1, Dermis D1 und Subcutis S1. Gemäß dem Stand der Technik werden aus einem Hautentnahmeareal meist nur Bereiche der Spalthaut B1 herausgeschnitten oder herausgestanzt.

Wünschenswerter sind bei einer Hautentnahme für eine Transplantation Bereiche der Vollhaut B2, die auch Hautanhangsgebilde wie Haare, Schweißdrüsen A1, Talgdrüsen T1, Rezeptoren und Hautnerven enthalten können.

Wie eingangs erläutert, lässt sich mit dem erfindungsgemäßen Vorrichtungsset zu einer Erzeugung einer Mikrograftmatrix aus Vollhautstanzteilen 12 aus einer Haut eines Hautentnahmeareals ein weitaus größeres Transplantat ohne Narbenbildung erzielen. Bei einer Entnahme einer Vielzahl an Vollhautstanzteilen und bei geeigneten Abständen der Vollhautstanzteile zueinander ein Spenderareal von 2- 3% der Körperoberfläche, wie im Stand der Technik, auf nunmehr 20- 50% erweitern.

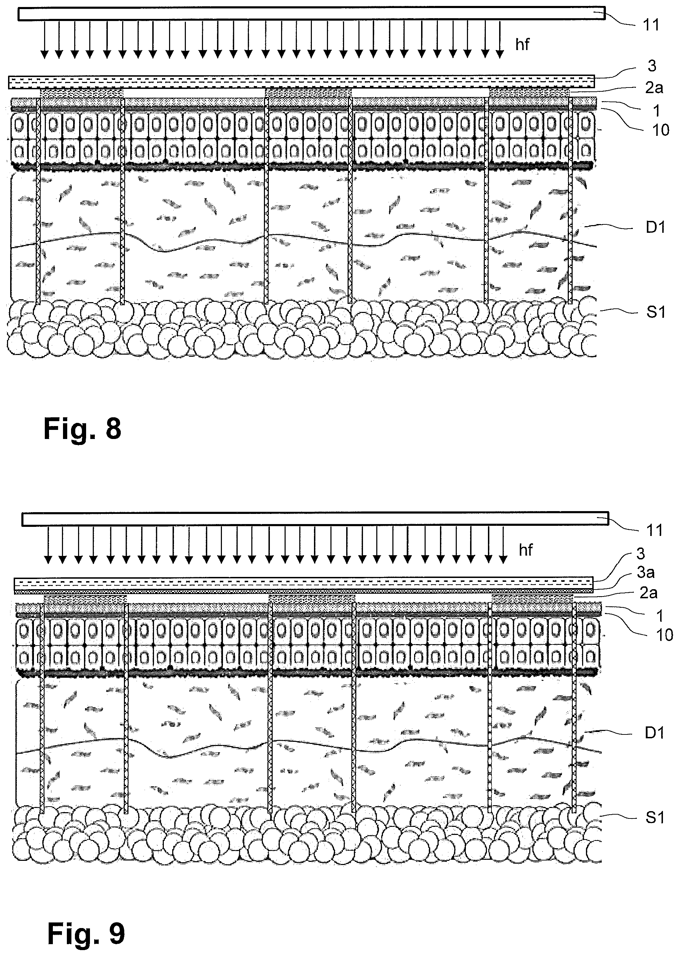

Das erfindungsgemäße Vorrichtungsset zur Erzeugung der Mikrograftmatrix umfasst generell folgende Komponenten:
a) ein Folienset mit einer vorbestimmten Dicke aus mindestens einer ersten Folie 1 und einer zweiten Folie 2. Dabei ist die erste Folie 1 flächig ausgebildet, um mit einer flächigen ersten Unterseite auf die Haut geklebt zu werden. Eine der ersten Unterseite gegenüberliegende erste Oberseite ist koplanar zu und haftet an einer zweiten Unterseite der zweiten Folie 2 mit einer zweiten Klebefestigkeit, wobei die zweite Folie 2 aber von der ersten Folie 1 abziehbar ist.
b) ein Klebemittel 10 zum Aufkleben der ersten Unterseite der ersten Folie 1 auf die Haut, wobei zwischen der ersten Unterseite der ersten Folie 1 und der Haut eine erste Klebefestigkeit erzeugt wird. Die erste Klebefestigkeit ist größer als die zweite Klebefestigkeit, so dass die zweite Folie 2 von der auf der Haut klebenden ersten Folie 1 abziehbar ist.
   Als Klebefestigkeit oder Haftfestigkeit wird generell eine physikalische Größe gesehen, die in N/cm² angegeben wird. Seitliche Reibkräfte der Vollhautstanzteile in der Haut, die seitlich zylinderförmig geschnitten sind, können dabei auch eine Komponente zur jeweiligen Klebe- oder Haftfestigkeit liefern.
c) eine Schneidevorrichtung mit einem Adapter, wobei der Adapter ausgebildet ist, durch Anpressen an das Folienset, das auf die Haut geklebt ist, unter Berücksichtigung der Dicke des Foliensets im Hautentnahmeareal einen vordefinierten Abstand zur darunter liegenden Haut herzustellen. Der vorbestimmte Abstand des Adapters zur Haut, wobei dabei die Hautoberfläche gemeint ist, entspricht bevorzugt der Dicke des Foliensets. Das Folienset wird bei üblichen geringen Anpressdrucken auf die Haut im Wesentlichen als inkompressibel angenommen, so dass es seine Dicke behält. Der Anpressdruck kann dabei in einem Bereich von 0,01 - 1 N/cm² oder höher liegen.
   Die Schneidvorrichtung ist dabei ausgebildet, eine Vielzahl an zueinander parallelen hohlzylindrischen Schnitten mit jeweils einem Hohlzylinderinnendurchmesser und einem Hohlzylinderabstand zueinander senkrecht zum Folienset und bis zu einer vorbestimmten Tiefe in der darunterliegenden Haut zu erzeugen. Dabei werden die Vielzahl der hohlzylindrischen Schnitte matrixförmig über das Hautentnahmeareal verteilt erzeugt. Dabei haben die hohlzylindrischen Schnitte, die senkrecht zum Folienset und in die Haut hinein erzeugt werden zu den benachbarten Schnitten den jeweiligen Hohlzylinderabstand, der zwischen den Schnitten auch variieren kann. Beispielsweise können runde Schnittlinien, die auf dem Folienset und auf der Hautoberfläche durch die hohlzylindrischen Schnitte entstehen, schachbrettmusterartig oder wabenartig oder anders verteil sein. Durch die hohlzylindrischen Schnitte, oder nur kurz "Schnitte" genannt, wird das Folienset in jeweilige innere Abschnitte und einen jeweiligen äußeren Abschnitt unterteilt. Die zweite Folie wird dabei in jeweilige innere Abschnitte 2a, die jeweils über dem jeweiligen Vollhautstanzteil 12 liegen, und in einen äußeren Abschnitt 2b unterteilt, aus dem die inneren Abschnitte 2a ausgeschnitten sind. Da die inneren Abschnitte 2a voneinander beanstandet sind, bleibt der äußere Abschnitt 2b der zweiten Folie zusammenhängend.
d) eine dritte Folie 3 mit einer Größe, die zumindest so groß wie das Entnahmeareal ist. Dabei ist die dritte Folie 3 ausgebildet ist, in Kontakt mit einer zweiten Oberseite der zweiten Folie 2, die der zweiten Unterseite der zweiten Folie 2 gegenüberliegt, eine dritte Klebefestigkeit zu erzeugen. Dabei kann die dritte Klebefestigkeit durch einfachen Kontakt der Folien oder der jeweiligen Oberflächen der Folien miteinander erzeugt werden, oder beispielsweise mit Hilfe einer ersten Lichtstrahlung hf einer ersten Lichtquelle 11, wie in den Figuren 8 und 9 gezeigt. Alternativ kann die Verklebung oder die dritte Klebefestigkeit durch eine Vernetzung und/oder Adhäsion der zweiten Folie 2 mit der dritten Folie 3 erfolgen. Dazu sind die zweite Folie 2 und die dritte Folie 3 entsprechend ausgebildet, eine solche Vernetzung oder Adhäsion miteinander zu erzeugen.
   Wichtig ist dabei, dass die erste, die zweite und die dritte Klebefestigkeit größer als eine vierte Haftfestigkeit ist, mit der die geschnittenen Vollhautstanzteile 12 in der Haut gehalten werden. Die vierte Haftfestigkeit wird teils durch eine seitliche Reibung des jeweiligen Vollhautstanzteils 12 in der Haut und teils durch die Haftung des jeweiligen Vollhautstanzteils 12 an einem Bodenende, das nicht durchschnitten ist, erzeugt. Die vierte Haftfestigkeit kann auch als Hebefestigkeit verstanden werden, die notwendig ist das Vollhautstanzteil aus der Haut zu heben. Da die erste 1, zweite 2 und dritte Folie 3 und das Klebemittel 10 so ausgebildet sind, dass die erste, zweite und dritte Klebefestigkeit größer als die vierte Haftfestigkeit ist, lassen sich die geschnittenen Vollhautstanzteile 12 aus der Haut ziehen, die eine Spenderhaut ist.

In Fig. 2 ist ein seitliches Schnittbild der Haut mit Epidermis E1, Dermis D1, die aus einer papillaren und einer retikulären Dermis besteht, und Subcutis S1 dargestellt. Auf der Haut ist das Folienset aus der ersten Folie 1 und der zweiten Folie 2 aufgeklebt. Dazwischen ist die Klebeschicht 10 zu sehen.

Bevorzugt weist die zweite Folie 2 ein Abziehmittel 9 oder eine Lasche auf, beispielsweise als einen überstehenden oder abstehenden Abschnitt eines äußeren Randbereichs des äußeren Abschnitts 2b der zweiten Folie 2. Dabei kann das Abziehmittel 9 oder die Lasche integraler Bestandteil der zweiten Folie 2 oder eine darauf aufgeklebte weitere Schicht sein, um die zweite Folie 2 damit halten und von der ersten Folie 1 abziehen zu können.

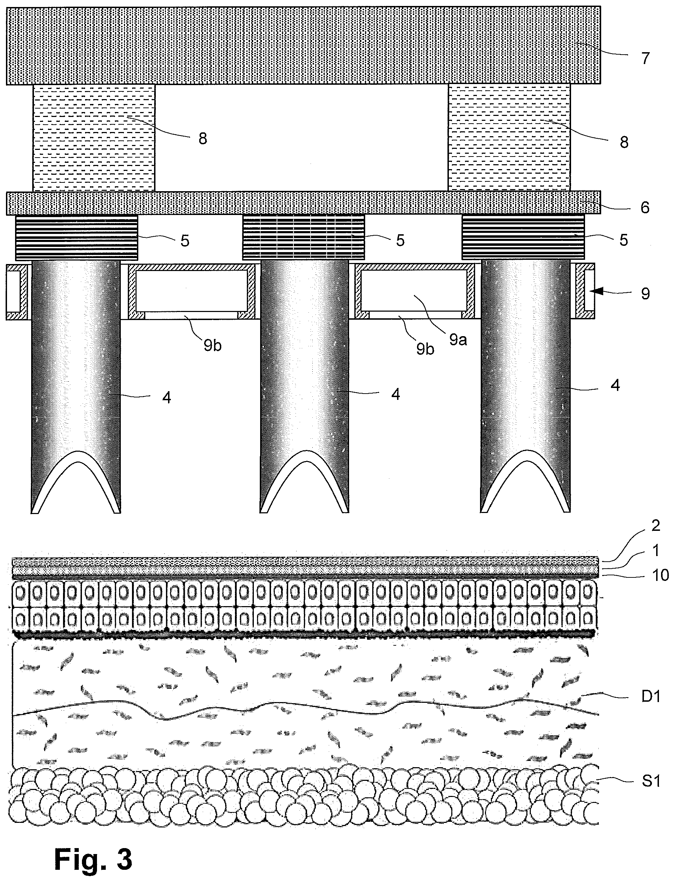

In Fig. 3 ist die Anordnung der Haut mit dem aufgeklebten Folienset aus Fig. 2 mit einer bevorzugten Schneidevorrichtung skizziert. Die bevorzugte Schneidevorrichtung ist dabei mit einem Trägerelement 6 und einer Vielzahl an Mikrohohlstanzen 4 ausgebildet, wobei die Mikrohohlstanzen 4 jeweils entlang einer Längsachse und an einem unteren Ende mit einer Schneidekante ausgebildet sind und jeweils einen röhrenförmigen Hohlraum aufweisen. Die Mikrohohlstanzen 4 können auch als Hohlnadeln bezeichnet werden. Bei einem senkrechten Einfahren in die Haut können dabei also die hohlzylindrischen Schnitte erzeugt werden. Die Mikrohohlstanzen 4 sind mit einem oberen der Schneidekante gegenüberliegenden Abschnitt mit dem Trägerelement 6 so verbunden, dass sie parallel zueinander eine matrixförmige Anordnung der Mikrohohlstanzen 4 bilden. Dabei weisen die Mikrohohlstanzen 4 eine Steifigkeit, einen Durchmesser und eine Länge auf, dass sie sich durch das Folienset hindurch bis zur vorbestimmten Tiefe hin erstrecken und sich in die Haut drücken lassen, ohne zu brechen oder abzuknicken. Der Adapter ist dazu bevorzugt rutschfest mit dem Hautentnahmeareal verbunden.

Generell ist mit der vorbestimmten Tiefe, mit der in die Haut geschnitten wird, diejenige Tiefe gemeint, die sich unter der ersten Folie 1 von der Hautoberfläche in die Tiefe der Haut erstreckt. Bei dem Erzeugen der Schnitte in die Tiefe der Haut durch die Schneidevorrichtung wird die Dicke des Foliensets aus erster 1 und zweiter Folie 2 berücksichtigt.

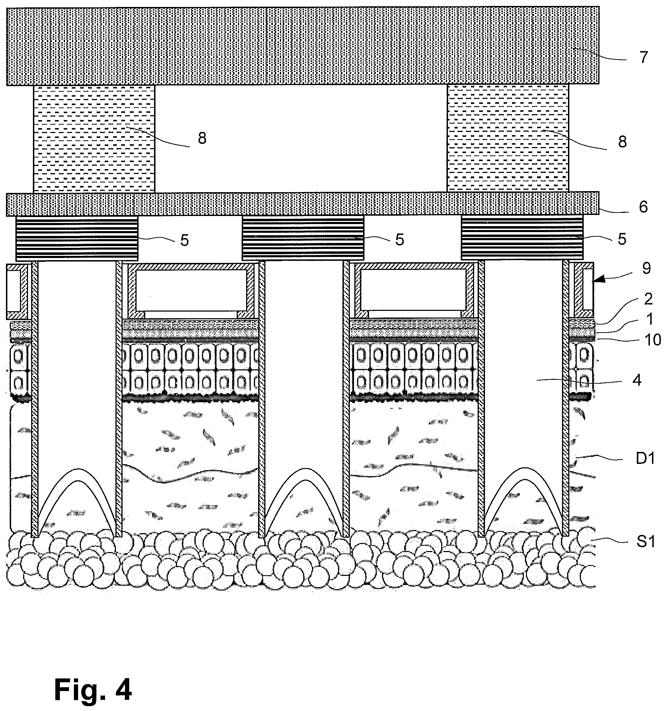

Die Mikroholstanzen 4 schneiden in einem vorderen Abschnitt also mit einer Länge der vorbestimmten Tiefe in die Haut ein. In Fig. 4 ist die Anordnung aus Fig. 3 in einem Zustand dargestellt, in dem die Mikrohohlstanzen 4 in die Haut bis zur vorbestimmten Tiefe eingefahrenen sind.

Bevorzugt weisen die Mikroholstanzen 4 in einem vorderen Abschnitt mit einer Länge der vorbestimmten Tiefe, der in die Haut einschneidet, eine Klingenwandstärke von 5 - 100 µm oder 50 - 200 µm auf.

In Fig. 3 und 4 wird das Trägerelement 6 als bevorzugte Ausführungsform zudem über einen weiteren Verschiebeaktuator 8 von einem zweiten Träger 7 gehalten.

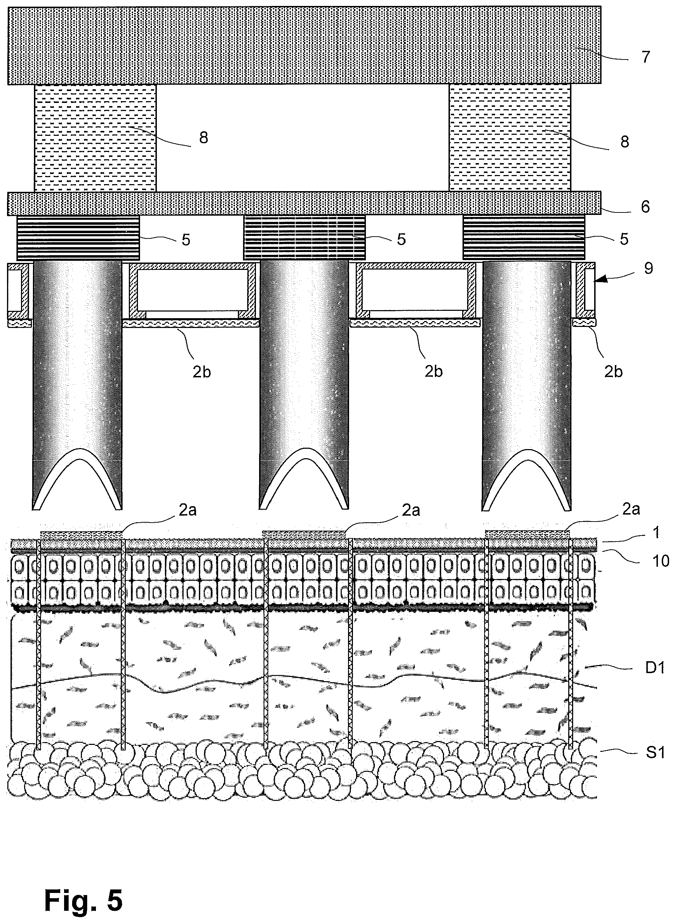

In Fig. 5 ist die Anordnung aus Fig. 3 und 4 in einem Zustand dargestellt, in dem die Mikrohohlstanzen 4 wieder aus der Haut herausgezogen sind. Dabei hält das Abziehmittel 9 den äußeren Abschnitt 2b der zweiten Folie 2 fest und zieht diesen mit ab, wobei die inneren Abschnitte 2a der zweiten Folie 2 auf der ersten Folie 1 zurückbleiben. Die verbleibenden Schnitte in der Haut sind auch zu erkennen. In Fig. 6 ist das Schnittbild der Haut mit der ersten 1 und zweiten Folie 2 nach dem Schneiden dargestellt, wobei der äußere Abschnitt 2b der zweiten Folie 2 nicht abgezogen ist. In Fig. 7 ist das Schnittbild der Haut mit der ersten 1 und zweiten Folie 2 nach dem Schneiden dargestellt, wobei der äußere Abschnitt 2b der zweiten Folie 2 teilweise schräg abgezogen ist. F1 gibt dabei die Abzugsrichtung an.

Alternativ zu den Mikrohohlstanzen 4 kann die Schneidevorrichtung auch einen steuerbaren Laser mit einer steuerbaren Optik umfassen, um einen solchen Laserstrahl erzeugen, der die Vielzahl der hohlzylindrischen Schnitte mit der vorbestimmten Tiefe durch das Folienset hindurch in die Haut hinein erzeugt. Bevorzugt weist der Laser eine Lichtwellenlänge auf im Bereich von

700 - 10 000 nm und ist bevorzugt ein IR-Laser bzw. ein Femtosekunden-Laser. Bevorzugt wird ein Laser aus dem Stand der Technik verwendet, der kalt schneidet, ohne dabei eine Koagulation oder Verklebung deines geschnittenen Gewebes zu erzeugen.

Bevorzugt sind die die erste Folie 1 und/oder die zweite Folie 2 dazwischen mit einer Photo-Polymerisationsschicht ausgebildet ist, die bei Bestrahlung mit der zweiten Lichtstrahlung die zweite Klebefestigkeit verstärkt. Dabei ist bevorzugt die Schneidevorrichtung mit einer zweiten Lichtquelle zur Erzeugung der zweiten Lichtstrahlung ausgebildet. Im Falle, dass die Schneidevorrichtung die Mikrohohlstanzen 4 umfasst, wird die zweite Lichtstrahlung bevorzugt durch den jeweiligen Hohlraum der jeweiligen Mikrohohlstanze 4 in Richtung der Haut geleitet. Beispielsweise kann die zweite Lichtstrahlung ausgehend von der zweiten Lichtquelle durch ein Bündel von Lichtleitern zu den jeweiligen Hohlräumen geleitet werden. Dadurch werden gezielt inneren Abschnitte 2a der zweiten Folie 2 bestrahlt, ohne den äußeren Abschnitt 2b der zweiten Folie 2 zu bestrahlen.

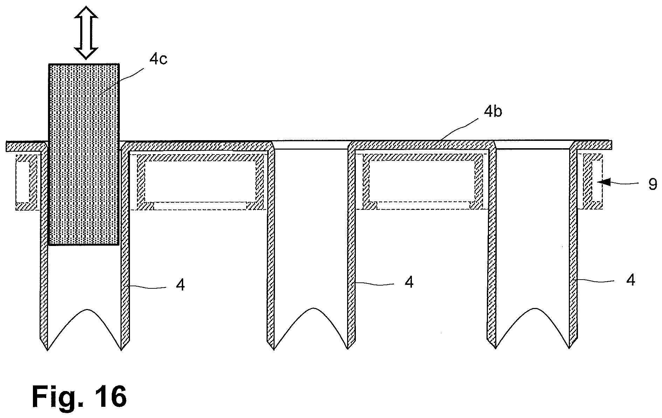

Bevorzugt sind in der Schneidevorrichtung, wie in Fig. 16 dargestellt, eine Vielzahl an Mandrins 4c so angeordnet, dass sie in den jeweiligen Mikrohohlstanzen 4 verfahrbar oder verschieblich sind. Dabei haben sie einen Außendurchmesser, der etwas kleiner als der Innendurchmesser der Mikrohohlstanzen 4 ist um nicht zu klemmen. Dabei sind die Mandrins 4c bevorzugt in Bezug zu den Mikrohohlstanzen 4 entlang der jeweiligen Längsachse gesteuert verfahrbar oder verschieblich. Die Ansteuerung erfolgt dabei bevorzugt so, dass die Mandrins 4c bei einem In-die-Haut-Drücken der Schneidevorrichtung den jeweiligen Hohlraum in den Mikrohohlstanzen 4 freigeben, damit das jeweilige Vollhautstanzteil 12 darin eindringen kann, wenn die Mikrohohlstanze in die Haut gedrückt wird. Bevorzugt werden vor einem Herausziehen der Mikrohohlstanzen 4 aus der Haut die Mandrins 4c bis zur Haut des jeweiligen Vollhautstanzteils 12 vorgefahren, wenn sie sich nicht schon dort befinden. Beim Herausziehen der Mikrohohlstanzen 4 werden die Mandrins 4c relativ zur restlichen Haut und dem Adapter konstant gehalten oder eher noch etwas weiter in die Haut vorgeschoben, um die jeweiligen Vollhautstanzteile 12 in der Haut zurückzuhalten.

Bevorzugt umfasst die Schneidevorrichtung zusätzlich ein geschlossenes Fluidsystem mit einem Behälter und einem steuerbaren Verschiebekolben, der durch eine Steuerung ansteuerbar ist, um ein Verschiebevolumen zu erzeugen. In dem Fluidsystem befindet sich eine Flüssigkeit, die in einem ersten Schritt durch die Ansteuerung und den Verschiebekolben in die jeweiligen Mikrohohlstanzen 4 bis mindestens zur jeweiligen Schneidekante hin verschoben wird. In einem zweiten und dritten Schritt, wenn die Mikrohohlstanzen 4 in die vorbestimmte Tiefe der Haut hinein- und daraus herausgezogen werden, steuert die Steuerung den Verschiebekolben so an, dass während des zweiten und dritten Schritts der Pegelstand der Flüssigkeit in den Mikrohohlstanzen 4 in Bezug zum Adapter und der Hautoberfläche im Wesentlichen konstant gehalten wird, um die Vollhautstanzteile 12 beim Herausziehen 12 der Mikrohohlstanzen 4 aus der Haut in die Vollhautstanzteile 12 zurückzuhalten. Die Flüssigkeit in der Mikrohohlstanze erfüllt im Wesentlichen den gleichen Zweck, wie das alternative Mandrin. Die Flüssigkeit ist bevorzugt eine wässrige Flüssigkeit eines der folgenden Bestandteile oder eine Mischung davon: Wasser, Salz, Adrenalin, Lokalanästhetika, Wachstumsfaktoren, Vitamine, Coenzyme, oder ein anderes Medikament.

Bevorzugt ist die Schneidevorrichtung ausgebildet, indem die Mikrohohlstanzen 4 mit dem Trägerelement 6 über einen jeweiligen ersten Aktuator 5 verbunden sind. Dabei ist der jeweilige erste Aktuator 5 ausgebildet und ansteuerbar, um die jeweilige mit dem ersten Aktuator 5 verbundene Mikrohohlstanze 4 in Längsrichtung und/oder in einer rotatorischen Drehbewegung um die Längsachse schwingen zu lassen. Dabei erfolgt die Anregung mit einer ersten Schwingfrequenz und ersten Schwingamplitude in der Längsrichtung und/oder einer zweiten Schwingfrequenz und zweiten Schwingamplitude für die Drehbewegung, die jeweils so vorbestimmt sind, dass eine Adhäsion der Vollhautstanzteile 12 mit den Mikrohohlstanzen 4 weitgehend vermieden wird. Bevorzugt liegt die erste und die zweite Schwingfrequenz im Bereich von 20kHz bis 10 MHz. Bevorzugt liegt die erste und die zweite Schwingamplitude im Bereich von 1µm bis 300 µm. Die Aktuatoren 5 sind bevorzugt Piezoaktuatoren oder elektromagnetische Aktuatoren.

Bevorzugt ist die Schneidevorrichtung ausgebildet, dass das Trägerelement 6 über mindestens einen zweiten Aktuator 8 mit einem zweiten Träger verbunden ist, wobei der mindestens eine zweite Aktuator 8 ausgebildet und ansteuerbar ist, den ersten Träger 6 mit den Mikrohohlstanzen 4 in der Längsrichtung zwischen einer ersten und einer zweiten Stellung zu verfahren. In der ersten Stellung sind die Mikrohohlstanzen 4 bevorzugt unmittelbar vor der zweiten Folie angeordnet, ohne einzudringen. Alternativ dazu wird durch einen Sensor eine Berührung oder ein geringfügiges Eindringen der Mikrohohlstanzen 4 in das Folienset detektiert, wobei die Bewegung der Mikrohohlstanzen 4 in die Haut zunächst gestoppt wird. In der zweiten Stellung erreichen die Mikrohohlstanzen 4 mit ihren Schneiden die vorbestimmte Tiefe. Danach werden die Mikrohohlstanzen 4 durch den zweiten Aktuator 8 aus der Haut und aus dem Folienset gezogen. Bevorzugt ist der zweite Aktuator 8 ausgebildet, die Mikrohohlstanzen 4 mit einer ersten Geschwindigkeit in die Haut einzufahren und mit einer zweiten Geschwindigkeit herausgefahren werden. Bevorzugt liegt die erste Geschwindigkeit in einem Bereich von 10 - 500m/s. Bevorzugt werden die Mikrohohlstanzen 4 dabei so stark beschleunigt, dass eine Bruchenergie der Haut überwunden wird, die bei 19-24kJ/m² liegt. Bevorzugt ist die zweite Geschwindigkeit 1- 100 m/s. Bevorzugt ist der mindestens eine zweite Aktuator 8 ein Piezoaktuator oder ein elektromagnetischer Aktuator.

Bevorzugt dient eine Pinzette oder Zange oder Lasche zum Abziehen des äußeren Abschnitts2b von der ersten Folie, Bevorzugt wird der äußere Abschnitt 2b dabei durch das Abziehmittel 9 oder die Lasche gehalten. Alternativ kann das Vorrichtungsset eine ansaugende oder klebende Anordnung umfassen, die zwischen den Mikrohohlstanzen 4 über dem äußeren Abschnitt 2b der zweiten Folie 2 angeordnet und ausgebildet ist, den äußeren Abschnitt 2b durch Ansaugen oder damit-Verkleben anzuziehen und wegzuziehen. Fig. 3 zeigt eine Abziehvorrichtung 9, die von einem Hohlraum 9a mit damit verbundenen Löchern 9b durchzogen ist, wobei die Löcher 9b nach unten zum zweiten Abschnitt 2b der zweiten Folie hin ausgerichtet sind. Wenn der äußere Abschnitt 2b der zweiten Folie 2 angezogen werden soll, wird die Abziehvorrichtung 9 vor die Folie gefahren und der Hohlraum dann mit einem Unterdruck beaufschlagt, um den zweiten Abschnitt 2b der zweiten Folie 2 anzuziehen und dann bei einem Wegfahren der Abziehvorrichtung 9 von der ersten Folie 1 abziehen zu können. Bevorzugt dient die Abziehvorrichtung 9, wie in Fig. 3 dargestellt, auch als Anschlag des Adapters auf dem Folienset oder der Haut.

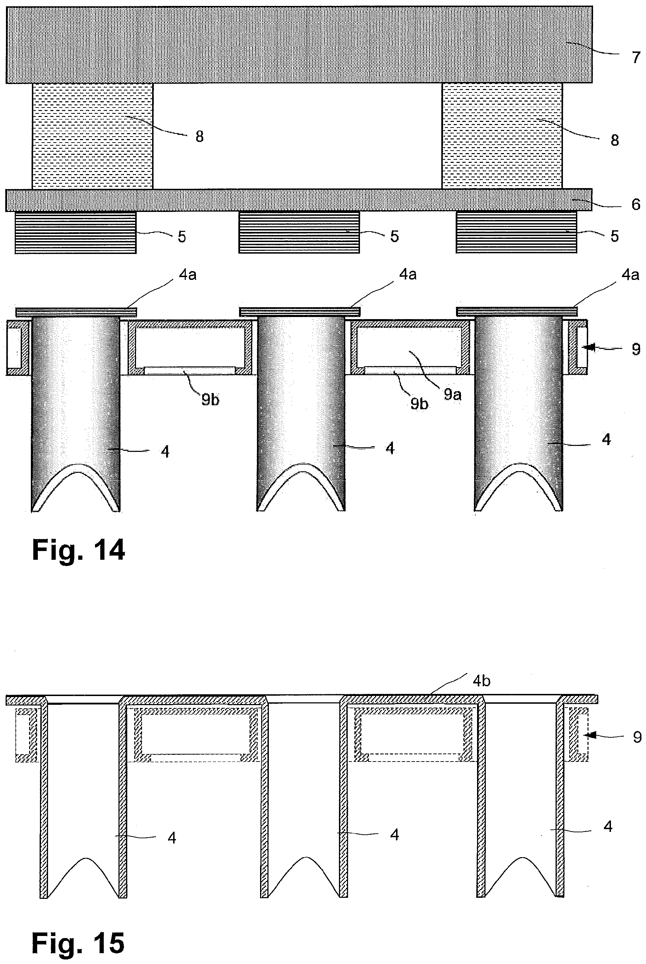

In Fig. 14 ist eine bevorzugte Ausbildungsform der Mikrohohlstanzen 4 dargestellt, die an dem jeweiligen Ende, das der Schneidekante gegenüberliegt, mit einer Kontaktstelle 4a ausgebildet sind. Dabei können die Mikrohohlstanzen 4 über die Kontaktstellen 4a mit jeweiligen korrespondierenden Gegenkontaktstellen am Trägerelement 6 verbunden und davon wieder gelöst werden. So können die Vielzahl der Mikrohohlstanzen 4 leicht am Trägerelement 6 ausgetauscht werden.

Alternativ bevorzugt ist, wie in Fig. 15 dargestellt, die Vielzahl der Mikrohohlstanzen 4 aus einem gemeinsamen Blech 4b herausgestanzt und gestülpt, wobei ein oberes den Schneidekanten gegenüberliegendes Blechende flächig plattenförmig ausgebildet ist und Löcher aufweist, die mit den röhrenförmigen Hohlräumen der Mikrohohlstanzen 4 korrespondieren. Dabei dient das obere Blechende als eine Kontaktstelle zu einer damit korrespondierenden Gegenkontaktstelle am Trägerelement 6. Dadurch ist die Vielzahl der Mikrohohlstanzen 4 eine Einheit und kann leicht mit dem Trägerelement 6 verbunden und wider davon gelöst werden.

Bevorzugt bestehen die Mikrostanzen 4 im Wesentlichen aus folgendem Material oder einem Gemisch oder einer Kombination davon: Titan, rostfreier Stahl, Faserverbundwerkstoff, ein biologisch abbaubarer Stoff, Poly-Lactid-co-Glycolid (PLGA), ein Sacharid, Polymere, Proteine, Spinnenseiden -Protein, Kollagen/Gelatine quervernetzt und ggf. mineralisiert, Cellulose.

Bevorzugt weisen die Mikrohohlstanzen 4 eine Monolayer-Beschichtung auf, die ausgebildet ist, um eine möglichst geringe Adhäsion mit der Haut zu bekommen. Bevorzugt weisen die Mikrohohlstanzen 4 eine Beschichtung folgender Art oder eine Kombination davon auf: Parylen, ASD (Atomic Single Layer Deposition), eine hydrophobe Beschichtung, eine polarisierte Beschichtung, die eine negativ geladene Oberfläche aufweist, eine SAM-Beschichtung (SAM = Self assambling Monolayer), eine Beschichtung mit einer Fluor-Verbindung oder Teflon.

Bevorzugt umfasst die Schneidevorrichtung eine Flüssigkeit und eine damit verbundene Pumpe, die mit den Hohlräumen der Mikrohohlstanzen 4 verbunden ist und die Flüssigkeit mit einer vorbestimmten Menge in die Mikrohohlstanzen 4 hinein oder zurück verschiebt. Bevorzugt umfasst die Flüssigkeit dabei eines oder eine Mischung von folgendem: Wasser, Salz, Adrenalin, Lokalanästhetika, Wachstumsfaktoren, Vitamine, Coenzyme, oder ein anderes Medikament.

In einer bevorzugten Ausführungsform weist die dritte Folie 3 mindestens eine Perforation auf, die jeweils ein Ausbruchareal umschließt und bildet. Dabei ist die mindestens eine Perforation so ausgebildet, dass das jeweilige Ausbruchareal der dritten Folie 3 daraus manuell oder maschinell ausgebrochen werden kann, nachdem die dritte Folie 3 mit den inneren Abschnitten 2a der zweiten Folie 2 und mit den Vollhautstanzteilen 12 verklebt ist. Dabei ist die mindestens eine Perforation ausgebildet, dass das jeweilige Ausbruchareal einen Durchmesser von bevorzugt 2- 10 mm aufweist. Nach einem Ausbrechen des mindestens einen Ausbruchareals oder der Folieninsel mit den Vollhautstanzteilen 12 kann die Folieninsel so als Makrograft beispielsweise zur Narbenbehandlung in ein entsprechend vorgestanztes Behandlungs-Hautareal transplantiert werden.

Bevorzugt ist die erste Folie 1 auf der ersten Oberseite zur zweiten Folie 2 hin mit einer Photo- Polymerisationsschicht ausgebildet, die unter Bestrahlung mit einer zweiten Lichtstrahlung eine anfängliche zweite Klebefestigkeit verstärkt, bis die zweite Klebefestigkeit erzeugt ist. Bevorzugt ist die zweite Unterseite der zweiten Folie 2 auf der Seite zur ersten Folie 1 hin mit einer Photo- Polymerisationsschicht ausgebildet ist, die unter Bestrahlung mit der zweiten Lichtstrahlung die zweite Klebefestigkeit verstärkt.

Bevorzugt sind die erste Folie 1 und/oder die zweite Folie 2 ausgebildet, Lichtanteile mit einer kürzeren Wellenlänge als 400nm um mindestens 50% zu dämpfen.

Bevorzugt umfassen die erste Folie 1 und/oder die zweite Folie 2 im Wesentlichen eines der folgenden Materialien: Polymerfolie, Silikonfolie, Polyethylen-Folie, PLGA-Folie.

Bevorzugt liegt die vorbestimmte Dicke des Foliensets in einem Bereich von 0,01 - 1 mm oder 1-3 mm.

Bevorzugt liegt eine erste Dicke der ersten Folie 1 in einem Bereich von 0,01 - 0,1 mm oder 0,1 - 1 mm.

Bevorzugt ist eine zweite Dicke der zweiten Folie 2 mindestens so groß ist, dass die dritte Folie 3 beim Auflegen auf die inneren Abschnitte 2a, nachdem der äußere Abschnitt 2b abgezogen worden ist, die erste Folie nicht berührt. Bevorzugt liegt die zweite Dicke im Bereich von 0,01 - 0,1 mm oder 0,1 - 0,8 mm.

Bevorzugt weist die erste Folie 1 bei einer abgezogenen zweiten Folie 2 an der ersten Oberseite eine Oberfläche auf, die mit der dritten Folie 3 möglichst keine Adhäsion oder eine Klebefestigkeit erzeugt, die kleiner als ein Zehntel der ersten Klebefestigkeit ist.

Bevorzugt ist das Klebemittel 10 als eine erste Klebemittelschicht ausgebildet und mit der ersten Folie 1 auf der Seite zur Haut hin angeordnet. Bevorzugt umfasst das Klebemittel 10 im Wesentlichen eines der folgenden Materialien oder eine Mischung davon: Komponentenkleber, organische und biologische Polymere oder Biotin und Streptavidin bei denen die Polymerisation chemisch oder thermisch oder photoaktivierend oder durch ionisierende Strahlung initiiert wird.

Bevorzugt liegt die vorbestimmte Tiefe von der Hautoberfläche in die Haut hinein in einem Bereich von 50 - 500 µm oder 0,5 - 1 mm oder 1-3 mm. Im Wesentlichen erstreckt sich die vorbestimmte Tiefe in die Haut hinein bis zum unteren Ende der Dermis D1, das als lockere und lose Verschiebeschicht an die Subcutis angrenzt.

Bevorzugt liegt die vierte Haftfestigkeit, mit der die Vollhautstanzteile 12 in der Spenderhaut gehalten werden, in einem Bereich von 0,08 - 4 N/cm². Die erste 1, zweite 2 und die dritte Folie 3 werden so eingestellt und ausgewählt oder beschichtet, dass die erste, zweite und dritte Klebefestigkeit kleiner als die vierte Haftfestigkeit ist.

Die Mikrohohlstanzen 4 weisen bevorzugt einen Hohlzylinderinnendurchmesser auf, der eine hohlzylindrische Form mit einem Innendurchmesser von 0,1 - 0,3 mm oder 0,3 - 0,5 mm oder 0,1 - 1 mm hat. Der Hohlzylinderabstand ist definiert als ein kleinster Abstand zweier benachbarter hohlzylindrischer Schnitte, wobei der Hohlzylinderabstand in einem Bereich liegt von 0,1 - 1 mm oder 1-3 mm.

Bevorzugt sind die erste Folie 1 und/oder die zweite Folie 2 und/oder die dritte Folie 3 biokompatibel ist. Bevorzugt sind dabei die erste Folie 1 und/oder die zweite Folie 2 und/oder die dritte Folie 3 ausgebildet, biologisch abbaubar zu sein.

Bevorzugt ist die erste Folie 1 und/oder die zweite Folie 2 dazwischen mit einer Photo-Polymerisationsschicht ausgebildet, die bei Bestrahlung mit der zweiten Lichtstrahlung die anfängliche zweite Klebefestigkeit verstärkt, um die zweite Klebefestigkeit zu bilden. Dabei ist die Schneidevorrichtung mit einer zweiten Lichtquelle zur Erzeugung der zweiten Lichtstrahlung ausgebildet, und es wird die zweite Lichtstrahlung bevorzugt auf die zweite Folie 2 appliziert, dass nur die inneren Abschnitte 2a der zweiten Folie 2 bestrahlt werden.

Bevorzugt sind die dritte Folie 3 und die zweite Oberseite der zweiten Folie 2 ausgebildet, die dritte Klebefestigkeit unter Einwirkung der ersten Lichtstrahlung hf von der ersten Lichtquelle 11 zu erzeugen. Bevorzugt ist die dritte Folie 3 ausgebildet, sich unter Einwirkung der ersten Lichtstrahlung hf von der ersten Lichtquelle 11 zu verhärten.

In Fig. 8 und 9 sind jeweils seitliche Schnittbilder der Haut mit der ersten Folie 1 und der zweiten Folie 2 dargestellt, von der der äußere Abschnitt 2b abgezogen worden ist, wobei die dritte Folie 3 auf die inneren Abschnitte 2a aufgelegt ist und mit der ersten Lichtstrahlung hf bestrahlt wird. In Fig. 9 ist dabei eine bevorzugte zweite Klebeschicht 3a zwischen den inneren Abschnitten 2a der zweiten Folie 2 und der dritten Folie 3 angeordnet.

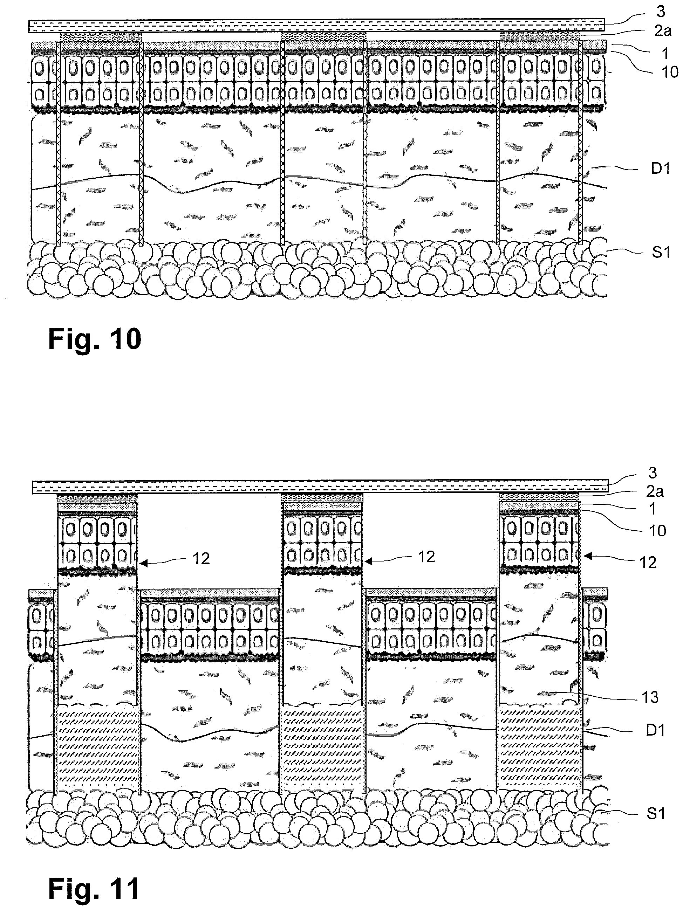

Fig. 10 zeigt ein seitliches Schnittbild der Haut mit der durch das Klebemittel 10 aufgeklebten ersten Folie 1, wobei auf der ersten Folie 1 in den Bereichen der Vollhautstanzteilen 12 die inneren Abschnitte 2a aufgeklebt sind, auf denen wiederum die dritte Folie 3 aufgeklebt ist.

Fig. 11 zeigt das seitliche Schnittbild der gleichen Komponenten wie in Fig. 10, jedoch sind die Vollhautstanzteile 12 durch die dritte Folie 3 teilweise herausgezogen.

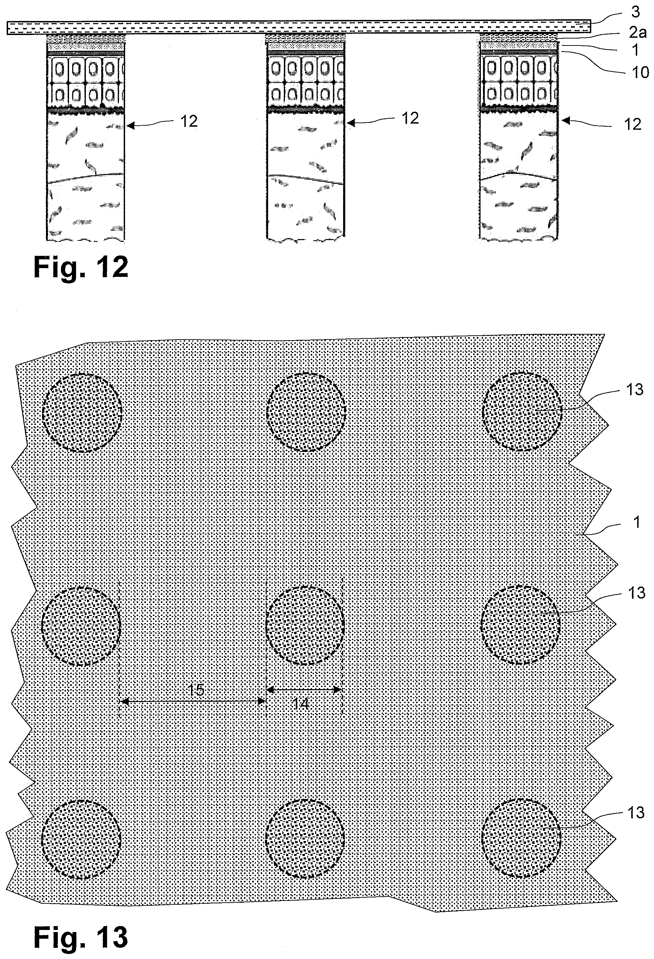

Fig. 12 zeigt die dritte Folie 3 mit den daran haftenden Vollhautstanzteilen 12 als die Mikrograftmatrix, nachdem die dritte Folie 3 von der Haut ganz abgezogen worden ist. Die Mikrograftmatrix kann nun zur Transplantation verwendet werden. Bevorzugt wird die Mikrograftmatrix noch mit flüssigem Kollagen und/oder anderen Medikamenten benetzt, bevor sie transplantiert wird.

Fig. 13 zeigt eine Teilansicht von oben des Hautentnahmeareals mit der ersten Folie 1, die auf der Haut klebt, wobei der äußere Abschnitt 2b der zweiten Folie 2 abgezogen ist und die dritte Folie 3 mit den daran haftenden Vollhautstanzteilen 12 auch abgezogen ist, so dass an den Stellen, wo die Vollhautstanzteile herausgezogen sind, Kavitäten 13 erzeugt und sichtbar sind. Die hohlzylindrischen Schnitte in die Haut sind auf der Hautoberfläche als Querschnitt mit einemjeweiligen Hohlzylinderinnendurchmesser 14 gestrichelt zu erkennen, wobei die hohlzylindrischen Schnitte eine jeweiligen Hohlzylinderabstand 15 aufweisen.

Bevorzugt weist die dritte Folie 3 zumindest auf ihrer Unterseite, die zur zweiten Oberseite der zweiten Folie 2 weist, eine photoaktivierbare Polymerschicht auf, die bei der Bestrahlung mit der ersten Lichtstrahlung hf mit der zweiten Folie 2 eine kovalente und/oder vernetzende Verbindung eingeht.

Bevorzugt weist die zweite Folie 2 zumindest auf ihrer Oberseite eine photoaktivierbare Polymerschicht auf, die bei der Bestrahlung mit der ersten Lichtstrahlung hf mit der dritten Folie 3 eine kovalente und/oder vernetzende Verbindung eingeht.

Bevorzugt umfasst die dritte Folie 3 im Wesentlichen eines der folgenden Materialien oder ein Gemisch davon: Polymerfolie, organische Polymere, biologische Polymere.

Die erste 1, die zweite 2 und die dritte Folie 3 sind bevorzugt über das gesamte Hautentnahmeareal flächig und koplanar ausgebildet.

Die Klebefestigkeit einer jeweiligen Folie mit einer jeweiligen angrenzenden Folie kann durch entsprechende Oberflächenrauigkeiten und/oder durch eine entsprechende miteinander gleiche oder zueinander invertierte Polarisation einer jeweiligen Oberfläche erzeugt werden. Zudem können Vernetzer die Flächen mehr oder weniger miteinander binden und entsprechende Klebefestigkeiten erzeugen. Solche Prozesse und Verbindungen sind aus dem Stand der Technik hinlänglich bekannt. Um eine Adhäsion beispielsweise der dritten Folie an der ersten Oberfläche der ersten Folie zu reduzieren, kann auch eine Nanobeschichtung vorgenommen werden.

Bevorzugt ist auch eine Apparatur, die von oben Positionen der inneren Abschnitte 2a erkennt und dort nach dem Auflegen der dritten Folie 3 gezielt durch eine Laser-Licht- oder Licht-Applikation die dritte Klebeverbindung erzeugt, beispielsweise durch verschweißen der inneren Abschnitte 2a der zweiten Folie 2 mit der dritten Folie 3.

Das nicht zur Erfindung gehörende Verfahren zur Erzeugung der Mikrograftmatrix mit der Vielzahl an Vollhautstanzteilen 12 aus der Haut des Hautentnahmeareals, umfasst der Reihenfolge nach die folgenden Schritte:
a) Bereitstellen
   - des Foliensets aus mindestens der ersten Folie 1 und der zweiten Folie 2, wobei die erste Folie 1 ausgebildet ist, mit der flächigen ersten Unterseite auf die Haut geklebt zu werden. Die der ersten Unterseite gegenüberliegende koplanar erste Oberseite ist dabei koplanar mit der zweiten Unterseite der zweiten Folie 2 mit der zweiten Klebefestigkeit aneinanderzuhaftend verbunden, wobei die zweite Folie 2 von der ersten Folie 1 abziehbar ist; und
   - der vom Folienset separaten dritten Folie 3, die eine Größe aufweist, die zumindest so groß wie das Entnahmeareal ist und die ausgebildet ist, in Kontakt mit der zweiten Oberseite der zweiten Folie 2, die der zweiten Unterseite der zweiten Folie 2 gegenüberliegt, die dritte Klebefestigkeit zu erzeugen, wobei die erste, die zweite und die dritte Klebefestigkeit größer als die vorbestimmte vierte Haftfestigkeit eingestellt ist;
b) Aufkleben der ersten Unterseite der ersten Folie 1 auf die Haut mit dem Klebemittel 10 dazwischen, wobei zwischen der ersten Unterseite der ersten Folie 1 und der Haut die erste Klebefestigkeit erzeugt wird, die größer als die zweite Klebefestigkeit ist, so dass die zweite Folie 2 von der auf der Haut klebenden ersten Folie 1 abziehbar ist;
c) Positionieren der Schneidevorrichtung mit dem Adapter auf dem Hautentnahmeareal, so dass durch den Adapter über dem Folienset der vordefinierte Abstand zur darunter liegenden Haut hergestellt wird.
d) Dann folgt das Schneiden der Vielzahl der zueinander parallelen hohlzylindrischen Schnitte, die jeweils den Hohlzylinderinnendurchmesser und einen oder verschiedene Hohlzylinderabstände zueinander haben, wobei die Schnitte senkrecht zum Folienset bis zur vorbestimmten Tiefe in der darunterliegenden Haut vorgenommen werden. Dabei sind die Vielzahl der Schnitte matrixförmig im Hautentnahmeareal verteilt und die jeweiligen Schnitte weisen einen Abstand voneinander auf, so dass sie sich nicht gegenseitig überschneiden. Dadurch wird das Folienset in eine Vielzahl innerer Abschnitte und einen jeweiligen äußeren Abschnitt unterteilt. Die zweite Folie wird dabei in die Vielzahl der inneren Abschnitte 2a und in einen äußeren Abschnitt 2b unterteilt, die inneren Abschnitte 2a der zweiten Folie 2 liegen dabei über dem jeweiligen geschnittenen Vollhautstanzteil 12. Dabei klebt der jeweilige innere Abschnitt 2a auf einem anderen inneren Abschnitt der ersten Folie, der wiederum mit dem jeweiligen Vollhautstanzteil 12 verklebt ist. Dabei wird das jeweilige geschnittene Vollhautstanzteil 12 einesteils durch die Hautverbindung am jeweiligen Bodenteil zur Subcutis hin und andernteils durch eine seitliche Reibung der zylindrischen Schnittfläche in der Haut gehalten, woraus die vierte Haftfestigkeit resultiert;
e) Abziehen des äußeren Abschnitts 2b der zweiten Folie 2 von der ersten Folie 1, wobei die inneren Abschnitte 2a der zweiten Folie 2 auf der ersten Folie 1 zurückbleiben;
f) Auflegen der dritten Folie 3 auf die Oberseiten der inneren Abschnitte 2a der zweiten Folie 2 und Andrücken der dritten Folie 3 darauf;
g) Verkleben der dritten Folie 3 mit den inneren Abschnitten 2a der zweiten Folie 2, wobei dazwischen eine dritte Klebefestigkeit erzeugt wird;
h) Abziehen der dritten Folie 3 von der Haut, wobei die Vielzahl der Vollhautstanzteile 12 als die Mikrograftmatrix mit aus der Haut gezogen werden und an der dritten Folie 3 kleben.

Bevorzugt erfolgt das Verkleben der dritten Folie 3 mit den inneren Abschnitten 2a der zweiten Folie 2 unter Einwirkung der ersten Lichtstrahlung hf der ersten Lichtquelle 11.

Bevorzugt wird die vorbestimmte Tiefe vor dem Schritt des Schneidens als diejenige Tiefe bestimmt, die sich im Wesentlichen bis zum unteren Ende der Dermis D1 und/oder zum Anfang der Subcutis erstreckt.

Bevorzugt das erfolgt Schneiden der hohlzylindrischen Schnitte in die Haut durch Verwendung einer der zuvor beschriebenen bevorzugten Ausführungsformen der Schneidevorrichtung.

Bevorzugt wird die Mikrograftmatrix nach dem Abziehen der dritten Folie 3 aus der Haut mit flüssigem Kollagen und/oder einem anderen Medikament benetzt oder gebadet.

Zur Klarheit werden unter den Merkmalen "oben", "unten", "Oberseite", Unterseite" relative Ortsangaben in senkrechter Richtung verstanden, so wie in den Figuren dargestellt. Seitlich bedeutet dabei horizontal, wie in den Figuren dargestellt. Unter dem Wortlaut "verfahrbar" wird verstanden, dass ein Vorrichtungsteil oder eine Vorrichtung gesteuert in seiner Lage bewegbar oder positionierbar ist.

Weitere mögliche Ausbildungsformen sind in den folgenden Ansprüchen beschrieben. Insbesondere können auch die verschiedenen Merkmale der oben beschriebenen Ausführungsformen miteinander kombiniert werden, soweit sie sich nicht technisch ausschließen.

Die In den Ansprüchen genannten Bezugszeichen dienen nur der besseren Verständlichkeit und beschränken die Ansprüche in keiner Weise auf die in den Figuren dargestellten Formen.

### Bezugszeichenliste

- A1: Schweißdrüse
- B1: Spalthaut
- B2: Vollhaut
- D1: Dermis
- E1: Epidermis
- F1: Abzugsrichtung der dritten Folie
- hf: Lichtstrahlung
- S1: Subcutis
- T1: Talgdrüse
- 1: erste Folie des Foliensets
- 2: zweite Folie des Foliensets
- 2a: innerer Abschnitt (der zweiten Folie)
- 2b: äußerer Abschnitt (der zweiten Folie)
- 3: dritte Folie
- 3a: zweite Klebeschicht
- 4: Mikrohohlstanze (bevorzugt zylindrisch röhrenförmig - auch: Mikroklinge)
- 4a: Kontaktstelle der Mikrohohlstanze
- 4b: Blech
- 4c: Mandrin (Stößel)
- 5: Aktuator (bevorzugt für longitudinale und rotatorische Bewegung)
- 6: Trägerelement
- 7: zweiter Träger
- 8: Verschiebeaktuator (in longitudinaler Richtung)
- 9: Abziehmittel
- 9a: Hohlraum
- 9b: Loch
- 10: Klebemittel
- 11: erste Lichtquelle
- 12: Vollhautstanzteil
- 13: Kavität
- 14: Hohlzylinderinnendurchmesser
- 15: Hohlzylinderabstand zueinander

## Patentansprüche

1. Vorrichtungsset zur Erzeugung einer Mikrograftmatrix mit einer Vielzahl an Vollhautstanzteilen (12) aus einer Haut eines Hautentnahmeareals, umfassend:
ein Folienset mit einer vorbestimmten Dicke aus mindestens einer ersten Folie (1) und einer zweiten Folie (2), wobei die erste Folie (1) ausgebildet ist, mit einer flächigen Unterseite auf die Haut geklebt zu werden und mit einer gegenüberliegenden Oberseite koplanar mit einer Unterseite der zweiten Folie (2) mit einer zweiten Klebefestigkeit aneinanderzuhaften und voneinander abziehbar zu sein;
ein Klebemittel (10) zum Aufkleben der Unterseite der ersten Folie (1) auf die Haut, wobei zwischen der Unterseite der ersten Folie (1) und der Haut eine erste Klebefestigkeit erzeugt wird, die größer als die zweite Klebefestigkeit ist, so dass die zweite Folie (2) von der auf der Haut klebenden ersten Folie (1) abziehbar ist;
eine Schneidevorrichtung mit einem Adapter, der ausgebildet ist, durch Anpressen an das Folienset, das auf die Haut geklebt ist, unter Berücksichtigung der Dicke des Foliensets im Hautentnahmeareal einen vordefinierten Abstand zur darunter liegenden Haut herzustellen, wobei die Schneidevorrichtung ausgebildet ist, eine Vielzahl an zueinander parallelen hohlzylindrischen Schnitten mit jeweils einem Hohlzylinderinnendurchmesser und einem Hohlzylinderabstand zueinander senkrecht zum Folienset und bis zu einer vorbestimmten Tiefe in der darunterliegenden Haut zu erzeugen, so dass die Vielzahl der hohlzylindrischen Schnitte matrixförmig im Hautentnahmeareal erzeugt wird, um durch die hohlzylindrischen Schnitte das Folienset in jeweilige innere Abschnitte mit einem jeweiligen inneren Abschnitt (2a) der zweiten Folie (2), der über dem jeweiligen geschnittenen Vollhautstanzteil (12) liegt, und in einen jeweiligen äußeren Abschnitt mit einem äußeren Abschnitt (2b) der zweiten Folie (2) zu unterteilen; und
eine dritte Folie (3) mit einer Größe, die zumindest so groß wie das Entnahmeareal ist und die ausgebildet ist, in Kontakt mit einer Oberseite der zweiten Folie (2), die der Unterseite der zweiten Folie (2) gegenüberliegt, eine dritte Klebefestigkeit zu erzeugen, wobei die erste, die zweite und die dritte Klebefestigkeit größer als eine vierte Haftfestigkeit ist, mit der die Vollhautstanzteile (12) in der Haut gehalten werden.

2. Vorrichtungsset gemäß Anspruch 1, wobei die Schneidevorrichtung ein Trägerelement (6) und eine Vielzahl an Mikrohohlstanzen (4) umfasst, die jeweils entlang einer Längsachse und an einem unteren Ende mit einer Schneidekante ausgebildet sind und einen röhrenförmigen Hohlraum aufweisen, um die dementsprechenden hohlzylindrischen Schnitte zu erzeugen, wobei die Mikrohohlstanzen (4) mit einem oberen der Schneidekante gegenüberliegenden Abschnitt mit dem Trägerelement (6) so verbunden sind, dass sie parallel zueinander eine matrixförmige Anordnung der Mikrohohlstanzen (4) bilden, wobei die Mikrohohlstanzen (4) eine Steifigkeit, einen Durchmesser und eine Länge haben, dass sie sich durch das Folienset hindurch bis zur vorbestimmten Tiefe erstrecken und in die Haut drücken lassen
wobei die Mikrohohlstanzen (4) in einem vorderen Abschnitt mit einer Länge der vorbestimmten Tiefe, der in die Haut einschneidet, eine Klingenwandstärke haben von 10 - 100µm oder 50 - 200 µm.

3. Vorrichtungsset gemäß Anspruch 2, wobei die erste Folie (1) und/oder die zweite Folie (2) dazwischen mit einer Photo-Polymerisationsschicht ausgebildet ist, die bei Bestrahlung mit der zweiten Lichtstrahlung die zweite Klebefestigkeit verstärkt, wobei die Schneidevorrichtung mit einer zweiten Lichtquelle zur Erzeugung der zweiten Lichtstrahlung ausgebildet ist und die zweite Lichtstrahlung so weitergeleitet wird, dass sie durch den inneren Hohlraum der jeweiligen Mikrohohlstanze (4) hindurch in Richtung zum Folienset hin erfolgt und dadurch nur die inneren Abschnitte (2a) der zweiten Folie (2) bestrahlt werden.

4. Vorrichtungsset gemäß einem der vorstehenden Ansprüche 2 oder 3, wobei die Schneidevorrichtung eine Vielzahl an Mandrins (4c) umfasst, die in den jeweiligen Mikrohohlstanzen (4) angeordnet sind, einen Außendurchmesser haben, der etwas kleiner als der Innendurchmesser der Mikrohohlstanzen (4) ist um nicht zu klemmen, und die in Bezug zu den Mikrohohlstanzen (4) entlang der jeweiligen Längsachse gesteuert verfahrbar sind, wobei die Mandrins (4c) beim In-die-Haut-Drücken der Schneidevorrichtung den jeweiligen Hohlraum in den Mikrohohlstanzen (4) freigeben, damit das jeweilige Vollhautstanzteil (12) darin eindringen kann, und die Mandrins (4c) bei einem Herausziehen der Mikrohohlstanzen (4) aus der Haut die Vollhautstanzteile (12) auf der Haut halten, wobei die Mikrohohlstanzen (4) von der Haut zurückgezogen werden und die Mandrins (4c) auf der Haut verbleiben.

5. Vorrichtungsset gemäß einem der vorstehenden Ansprüche 2-4, wobei die Schneidevorrichtung ein geschlossenes Fluidsystem mit einem Behälter, einem steuerbaren Verschiebekolben, der durch eine Steuerung angesteuert wird, und einer Flüssigkeit umfasst, die in einem ersten Schritt durch den Verschiebekolben in die jeweiligen Mikrohohlstanzen (4) bis mindestens zur jeweiligen Schneidekante hin verschoben wird, wobei in einem zweiten und dritten Schritt, wenn die Mikrohohlstanzen (4) in die vorbestimmte Tiefe der Haut hinein- und daraus herausgezogen werden, die Steuerung den Verschiebekolben so antreibt, dass während des zweiten und dritten Schritts der Pegelstand der Flüssigkeit in den Mikrohohlstanzen (4) in Bezug zur Haut im Wesentlichen konstant gehalten wird, um die Vollhautstanzteile (12) beim Herausziehen der Mikrohohlstanzen (4) aus der Haut in die Haut zurückgedrückt zu halten, wobei die Flüssigkeit eine wässrige Flüssigkeit ist, die eines oder eine Mischung aus folgendem umfasst: Wasser, Salz, Epinephrin, Wachstumsfaktoren, Vitamine, Coenzyme, oder ein anderes Medikament.

6. Vorrichtungsset gemäß einem der vorstehenden Ansprüche 2-5, wobei die Schneidevorrichtung ausgebildet ist, indem die Mikrohohlstanzen (4) mit dem Trägerelement (6) über einen jeweiligen ersten Aktuator (5) verbunden sind, wobei der jeweilige erste Aktuator (5) ausgebildet und ansteuerbar ist, um die jeweilige mit dem ersten Aktuator (5) verbundene Mikrohohlstanze (4) in Längsrichtung und/oder in einer rotatorischen Drehbewegung um die Längsachse schwingen zu lassen, wobei eine erste Schwingfrequenz und Schwingamplitude in der Längsrichtung und/oder eine zweite Schwingfrequenz und Schwingamplitude für die Drehbewegung so vorbestimmt ist, eine Adhäsion der Vollhautstanzteile (12) mit den Mikrohohlstanzen (4) weitgehend zu vermeiden, wobei die erste und die zweite Schwingfrequenz im Bereich von 20kHz bis 10 MHz liegen und die erste und die zweite Schwingamplitude im Bereich von 1µm bis 300 µm liegen; und/oder
wobei die ersten Aktuatoren (5) Piezoaktuatoren oder elektromagnetische oder andere Aktuatoren sind.

7. Vorrichtungsset gemäß einem der vorstehenden Ansprüche 2-6, wobei die Schneidevorrichtung ausgebildet ist, indem das Trägerelement (6) über mindestens einen zweiten Aktuator (8) mit einem zweiten Träger verbunden ist, und der mindestens eine zweite Aktuator (8) ausgebildet und ansteuerbar ist, den ersten Träger (6) mit den Mikrohohlstanzen (4) in der Längsrichtung zwischen einer ersten und einer zweiten Stellung zu verfahren, wobei die Mikrohohlstanzen (4) in der ersten Stellung noch vor der zweiten Folie angeordnet sind, ohne einzudringen, und in der zweiten Stellung in die vorbestimmte Tiefe bis im Wesentlichen zum Ende der Dermis (D1) eindringen, wobei die Mikrohohlstanzen (4) durch den zweiten Aktuator (8) in die Haut mit einer ersten Geschwindigkeit eingefahren und mit einer zweiten Geschwindigkeit herausgefahren werden; und/oder
wobei die zweiten Aktuatoren (8) Piezoaktuatoren oder elektromagnetische oder andere Aktuatoren sind.

8. Vorrichtungsset gemäß einem der vorstehenden Ansprüche 2-7, zudem ein Abziehmittel (9) oder eine Lasche als einen überstehenden oder abstehenden Abschnitt eines äußeren Randbereichs des äußeren Abschnitts (2b) der zweiten Folie (2) umfassend, wobei das Abziehmittel (9) oder die Lasche integraler Bestandteil der zweiten Folie (2) oder eine darauf aufgeklebte weitere Schicht ist, um die zweite Folie (2) daran halten und von der ersten Folie (1) abziehen zu können; oder
wobei das Abziehmittel (9) eine ansaugende oder klebende Anordnung ist, die zwischen den Mikrohohlstanzen (4) über dem äußeren Abschnitt (2b) der zweiten Folie (2) angeordnet und ausgebildet ist, den äußeren Abschnitt (2b) durch Ansaugen oder damit-Verkleben anzuziehen und wegzuziehen.

9. Vorrichtungsset gemäß einem der vorstehenden Ansprüche 2-8, wobei die Vielzahl der Mikrohohlstanzen (4) an dem jeweiligen Ende, das der Schneidekante gegenüberliegt, eine Kontaktstelle (4a) aufweisen, die über jeweilige korrespondierende Gegenkontaktstellen mit dem Trägerelement (6) verbunden sind, vom Trägerelement (6) trennbar und mit dem Trägerelement (6) verbindbar sind.

10. Vorrichtungsset gemäß einem der vorstehenden Ansprüche 2-8, wobei die Vielzahl der Mikrohohlstanzen (4) aus einem gemeinsamen Blech (4b) herausgestanzt und gestülpt sind, wobei ein oberes den Schneidekanten gegenüberliegendes Blechende flächig plattenförmig ist und Löcher aufweist, die mit den röhrenförmigen Hohlräumen der Mikrohohlstanzen (4) korrespondieren, wobei das obere Blechende eine Kontaktstelle zu einer damit korrespondierenden Gegenkontaktstelle zum Trägerelement (6) aufweist, wobei die Kontaktstelle mit dem Trägerelement (6) verbunden ist und vom Trägerelement (6) trennbar ist.

11. Vorrichtungsset gemäß einem der vorstehenden Ansprüche 2-10, wobei die Mikrostanzen (4) im Wesentlichen aus folgendem Material oder einem Gemisch oder eine Kombination daraus bestehen: Titan, rostfreier Stahl, Faserverbundwerkstoff, ein biologisch abbaubarer Stoff, Poly-Lactid-co-Glycolid (PLGA), ein Sacharid, Polymere, Proteine, Spinnenseide-Protein, Kollagen/Gelatine quervernetzt und ggf. mineralisiert, Cellulose; und/oder
wobei die Mikrohohlstanzen (4) eine Monolayer-Beschichtung aufweisen, die ausgebildet ist, um eine möglichst geringe Adhäsion mit der Haut zu bekommen; und/oder
wobei die Mikrohohlstanzen (4) eine Beschichtung folgender Art oder eine Kombination davon aufweisen: Parylen, ASD (Atomic Single Layer Deposition), eine hydrophobe Beschichtung, eine polarisierte Beschichtung, die eine negativ geladene Oberfläche aufweist, eine SAM-Beschichtung (SAM = Self assembling Monolayer), eine Beschichtung mit einer Fluor-Verbindung oder Teflon; und/oder
wobei die Schneidevorrichtung eine Flüssigkeit und eine damit verbundene Pumpe umfasst, die mit den Hohlräumen der Mikrohohlstanzen (4) verbunden ist und die Flüssigkeit mit einer vorbestimmten Menge in die Mikrohohlstanzen (4) hinein oder zurück verschiebt, wobei die Flüssigkeit eines oder eine Mischung von folgendem umfasst: Wasser, Salz, Epinephrin, Wachstumsfaktoren, Vitamine, Coenzyme, oder ein anderes Medikament.

12. Vorrichtungsset gemäß Anspruch 1, wobei die Schneidevorrichtung einen Laser mit einer Optik umfasst, um einen solchen Laserstrahl so erzeugen, der die Vielzahl der hohlzylindrischen Schnitte mit der vorbestimmten Tiefe durch das Folienset hindurch in die Haut hinein erzeugt; und/oder
wobei der Laser eine Lichtwellenlänge aufweist im Bereich von 700 - 10 000 nm und eines Typs IR-Laser und/oder ein Femtosekunden-Laser ist.

13. Vorrichtungsset gemäß einem der vorstehenden Ansprüche, wobei die erste Folie (1) auf der Seite zur zweiten Folie (2) hin mit einer Photo-Polymerisationsschicht ausgebildet ist, die unter Bestrahlung mit einer zweiten Lichtstrahlung die zweite Klebefestigkeit verstärkt; und/oder
wobei die zweite Folie (2) auf der Seite zur ersten Folie (1) hin mit einer Photo-Polymerisationsschicht ausgebildet ist, die unter Bestrahlung mit der zweiten Lichtstrahlung die zweite Klebefestigkeit verstärkt; und/oder
wobei die erste Folie (1) und/oder die zweite Folie (2) ausgebildet ist/sind, Lichtanteile mit einer kürzeren Wellenlänge als 400nm um mindestens 50% zu dämpfen; und/oder
wobei die erste Folie (1) und/oder die zweite Folie (2) im Wesentlichen aus einem der folgenden Materialien besteht/bestehen: Polymerfolie, Silikonfolie, Polyethylenfolie, PLGA-Folie; und/oder
wobei die vorbestimmte Dicke des Foliensets in einem Bereich von 0,01 - 1 mm oder 1 - 3 mm liegt; und/oder
wobei eine erste Dicke der ersten Folie (1) in einem Bereich von 0,01 - 0,1 mm oder 0,1 - 1 mm liegt; und/oder
wobei eine zweite Dicke der zweiten Folie (2) mindestens so groß ist, dass die dritte Folie (3) beim Auflegen auf die inneren Abschnitte (2a), nachdem der äußere Abschnitt (2b) abgezogen worden ist, die erste Folie nicht berührt; und/oder
wobei die zweite Dicke im Bereich von 0,01 - 0,1 mm oder 0,1 - 0,8 mm liegt; und/oder
wobei die erste Folie (1) bei einer abgezogenen zweiten Folie (2) eine der Haut gegenüberliegende obere Oberfläche aufweist, die mit der dritten Folie (3) eine Klebefestigkeit erzeugt, die kleiner als ein Zehntel der ersten Klebefestigkeit ist; und/oder
wobei das Klebemittel (10) als eine erste Klebemittelschicht ausgebildet und mit der ersten Folie (1) auf der Seite zur Haut hin angeordnet ist; und/oder
wobei das Klebemittel (10) im Wesentlichen aus einem der folgenden Materialien oder einer Mischung daraus besteht: Komponentenkleber, organische und biologische Polymere, bei denen die Polymerisation chemisch oder thermisch oder photoaktivierend oder durch ionisierende Strahlung initiiert wird;
wobei die vorbestimmte Tiefe von der Hautoberfläche in die Haut hinein in einem Bereich von 50 - 500 µm oder 0,5 - 1 mm oder 1 - 3 mm liegt; und/oder
wobei die vierte Haftfestigkeit, mit der die Vollhautstanzteile (12) in der Spenderhaut gehalten werden, in einem Bereich liegt von 0,08 - 4 N/cm²; und/oder
wobei der Hohlzylinderinnendurchmesser der hohlzylindrischen Form einen Innendurchmesser von 0,1 - 0,3 mm oder 0,3 - 0,5 mm oder 0,1 - 1 mm aufweist; und/oder
wobei der Hohlzylinderabstand definiert ist als ein kleinster Abstand zweier benachbarter hohlzylindrischer Schnitte und der Hohlzylinderabstand in einem Bereich von 0,1 - 1 mm oder 1 - 3 mm liegt; und/oder
wobei die erste Folie (1) und/oder die zweite Folie (2) und/oder die dritte Folie (3) biokompatibel ist; und/oder
wobei die erste Folie (1) und/oder die zweite Folie (2) und/oder die dritte Folie (3) ausgebildet ist, biologisch abbaubar zu sein.

14. Vorrichtungsset gemäß einem der vorstehenden Ansprüche, wobei die erste Folie (1) und/oder die zweite Folie (2) dazwischen mit einer Photo-Polymerisationsschicht ausgebildet ist, die bei Bestrahlung mit der zweiten Lichtstrahlung die zweite Klebefestigkeit verstärkt, wobei die Schneidevorrichtung mit einer zweiten Lichtquelle zur Erzeugung der zweiten Lichtstrahlung ausgebildet ist, wobei die zweite Lichtstrahlung so erzeugt wird, dass nur die inneren Abschnitte (2a) der zweiten Folie (2) bestrahlt werden.

15. Vorrichtungsset gemäß einem der vorstehenden Ansprüche, wobei die dritte Folie (3) und die Oberseite der zweiten Folie (2) ausgebildet sind, die dritte Klebefestigkeit unter Einwirkung einer ersten Lichtstrahlung (hf) von einer ersten Lichtquelle (11) zu erzeugen; und/oder
wobei die dritte Folie (3) ausgebildet ist, sich unter Einwirkung der ersten Lichtstrahlung (hf) von der ersten Lichtquelle (11) zu verhärten; und/oder
wobei die dritte Folie (3) zumindest auf ihrer Unterseite, die zur Oberseite der zweiten Folie (2) weist, eine photoaktivierbare Polymerschicht aufweist, die bei der Bestrahlung mit der ersten Lichtstrahlung (hf) mit der zweiten Folie (2) eine kovalente und vernetzende Verbindung eingeht; und/oder
wobei die zweite Folie (2) zumindest auf ihrer Oberseite eine photoaktivierbare Polymerschicht aufweist, die bei der Bestrahlung mit der ersten Lichtstrahlung (hf) mit der dritten Folie (3) eine kovalente und vernetzende Verbindung eingeht; und/oder
wobei die dritte Folie (3) im Wesentlichen aus einem der folgenden Materialien oder einem Gemisch daraus besteht: Polymerfolie, organische Polymere, biologische Polymere.

## Claims

1. A device set for generating a micrograft matrix with a plurality of punched parts of full-thickness skin (12) from skin of a skin removal area, comprising:
a film set having a predetermined thickness of at least one first film (1) and one second film (2),
wherein the first film (1) is adapted to be affixed to the skin with a flat bottom side and to adhere, with an opposite top side, to a bottom side of the second film (2) in a coplanar manner with a second adhesive strength and peelable from one another;
an adhesive (10) for affixing the bottom side of the first film (1) to the skin, wherein a first adhesive strength is created between the bottom side of the first film (1) and the skin, which is greater than the second adhesive strength, so that the second film (2) is peelable from the first film (1) affixed to the skin;
a cutting device having an adapter, which, by pressing it onto the film set affixed to the skin, considering the thickness of the film set in the skin removal area, is adapted to create a predefined distance to the subjacent skin, wherein the cutting device is adapted to produce a plurality of hollow cylindrical cuts in parallel to one another with a hollow-cylinder inner diameter and a hollow-cylinder distance from one another each vertical to the film set and down to a predetermined depth in the subjacent skin, so that the plurality of the hollow cylindrical cuts is produced in the form of a matrix in the skin removal area, in order to divide the film set into respective inner sections with a respective inner section (2a) of the second film (2) lying above the respectively cut punched part of full-thickness skin (12) and a respective outer section with an outer section (2b) of the second film (2) with the hollow cylindrical cuts; and
a third film (3) having a size, which is at least as large as the removal area and which is adapted to create a third adhesive strength in contact with a top side of the second film (2) opposite the bottom side of the second film (2), wherein the first, the second and the third adhesive strengths are greater than a fourth adherence, with which the punched parts of full-thickness skin (12) are held in the skin.

2. The device set according to claim 1, wherein the cutting device comprises a support element (6) and a plurality of micro hollow punches (4), which are respectively formed along a longitudinal axis and with a cutting edge at a lower end and having a tubular hollow space to produce the corresponding hollow cylindrical cuts, wherein the micro hollow punches (4) are connected with the support element (6) with an upper section opposite the cutting edge, such that they, in parallel to one another, form an arrangement of the micro hollow punches (4) in the form of a matrix, wherein the micro hollow punches (4) have such a stiffness, diameter and length, that they extend through the film set down to the predetermined depth and can be pressed into the skin,
wherein the micro hollow punches (4), in a front section with a length of the predetermined depth, which cuts into the skin, have a blade wall thickness of 10 - 100 µm or 50 - 200 µm.

3. The device set according to claim 2, wherein the first film (1) and/or the second film (2) are formed with a photopolymerization layer therebetween, which upon irradiation with the second light radiation enhances the second adhesive strength, wherein the cutting device is provided with a second light source for generating the second light radiation and the second light radiation is transferred such that it passes through the inner hollow space of the respective micro hollow punch (4) towards the film set and thus only the inner sections (2a) of the second film (2) are irradiated.

4. The device set according to one of the preceding claims 2 or 3, wherein the cutting device comprises a plurality of mandrins (4c), which are arranged in the respective micro hollow punches (4), have an outer diameter, which is slightly smaller than the inner diameter of the micro hollow punches (4), in order not to get stuck, and, relative to the micro hollow punches (4), can be controllably moved along the respective longitudinal axis, wherein the mandrins (4c), upon pressing the cutting device into the skin, release the respective hollow space in the micro hollow punches (4), so that the respective punched part of full-thickness skin (12) can be taken up therein, and the mandrins (4c), upon pulling the micro hollow punches (4) out of the skin, hold the punched parts of full-thickness skin (12) on the skin, wherein the micro hollow punches (4) are pulled back from the skin and the mandrins (4c) remain on the skin.

5. The device set according to one of the preceding claims 2-4, wherein the cutting device comprises a closed fluid system with a container, a controllable displacement piston controlled by a controller, and a liquid, which, in a first step, is displaced into the respective micro hollow punches (4) by the displacement piston at least up to the respective cutting edge, wherein, in a second and third step, when the micro hollow punches (4) are pressed down to the predetermined depth of the skin and pulled out therefrom, the controller drives the displacement piston such that, during the second and third step, the level of the liquid in the micro hollow punches (4) relative to the skin is substantially kept constant, in order to keep the punched parts of full-thickness skin (12) pressed back into the skin upon pulling the micro hollow punches (4) out of the skin, wherein the liquid is an aqueous liquid, which comprises one or a mixture of the following: water, salt, epinephrine, growth factors, vitamins, coenzymes, or another drug.

6. The device set according to one of the preceding claims 2-5, wherein the cutting device is formed by the micro hollow punches (4) being connected with the support element (6) via a respective first actuator (5), wherein the respective first actuator (5) is adapted and controllable to let the respective micro hollow punch (4) connected with the first actuator (5) vibrate in the longitudinal direction and/or in a rotational rotary movement around the longitudinal axis, wherein a first vibration frequency and vibration amplitude in the longitudinal direction and/or a second vibration frequency and vibration amplitude for the rotary movement are predetermined such that an adhesion of the punched parts of full-thickness skin (12) with the micro hollow punches (4) is largely avoided, wherein the first and the second vibration frequencies lie in the range from 20 kHz to 10 MHz and the first and the second vibration amplitudes lie in the range from 1 µm to 300 µm; and/or
wherein the first actuators (5) are piezo actuators or electromagnetic or other actuators.

7. The device set according to one of the preceding claims 2-6, wherein the cutting device is formed by the support element (6) being connected with a second support via at least one second actuator (8), and the at least one second actuator (8) is adapted and controllable to move the first support (6) with the micro hollow punches (4) in the longitudinal direction between a first and a second position, wherein, in the first position, the micro hollow punches (4) are still arranged above the second film, without penetrating it, and, in the second position, penetrate into the predetermined depth substantially down to the end of the dermis (D1), wherein the micro hollow punches (4) are inserted into the skin by the second actuator (8) with a first speed and retracted therefrom with a second speed; and/or
wherein the second actuators (8) are piezo actuators or electromagnetic or other actuators.

8. The device set according to one of the preceding claims 2-7, furthermore comprising a peeling means (9) or a tab as a projecting or protruding section of an outer edge region of the outer section (2b) of the second film (2), wherein the peeling means (9) or the tab is an integral component of the second film (2) or a further layer affixed thereon, in order to be able to hold the second film (2) therewith and peel it off from the first film (1); or
wherein the peeling means (9) is a sucking in or adhesive arrangement, which is arranged and formed between the micro hollow punches (4) above the outer section (2b) of the second film (2) for pulling on and peeling away the outer section (2b) by sucking it in or being affixed thereto.

9. The device set according to one of the preceding claims 2-8, wherein the plurality of micro hollow punches (4) respectively have a contact point (4a) at the respective end opposite the cutting edge, which via respective corresponding counter-contact points are connected with the support element (6), can be separated from the support element (6) and can be connected with the support element (6).

10. The device set according to one of the preceding claims 2-8, wherein the plurality of micro hollow punches (4) are punched out and inverted from a common metal sheet (4b), wherein an upper sheet end opposite the cutting edges is flat and plate-shaped and has holes, which correspond with the tubular hollow spaces of the micro hollow punches (4), wherein the upper sheet end has a contact point to a counter-contact point corresponding therewith on the support element (6), wherein the contact point is connected with the support element (6) and can be separated from the support element (6).

11. The device set according to one of the preceding claims 2-10, wherein the micro punches (4) substantially consist of the following material or a mixture or a combination thereof: titanium, stainless steel, fiber-reinforced composite, a biodegradable material, polylactic-co-glycolic acid (PLGA), a saccharide, polymers, proteins, spider silk proteins, collagen/gelatin, crosslinked and mineralized as appropriate, cellulose; and/or
wherein the micro hollow punches (4) have a monolayer coating adapted to get as little adhesion with the skin as possible; and/or
wherein the micro hollow punches (4) have a coating of the following type or a combination thereof: parylene, ASD (atomic single layer deposition), a hydrophobic coating, a polarized coating, having a negatively charged surface, a SAM coating (SAM = self-assembling monolayer), a coating with a fluorine compound or teflon; and/or
wherein the cutting device comprises a liquid and a pump connected therewith, which is connected with the hollow spaces of the micro hollow punches (4) and displaces the liquid at a predetermined amount into the micro hollow punches (4) or back therefrom, wherein the liquid comprises one or a mixture of the following: water, salt, epinephrine, growth factors, vitamins, coenzymes, or another drug.

12. The device set according to claim 1, wherein the cutting device comprises a laser with optics, in order to generate such a laser beam, that produces the plurality of hollow cylindrical cuts with the predetermined depth through the film set down into the skin; and/or
wherein the laser has a wavelength of light in the range of 700 - 10.000 nm and is an IR laser type and/or a femtosecond laser.

13. The device set according to one of the preceding claims, wherein the first film (1) is formed with a photopolymerization layer on the side towards the second film (2), which under irradiation with a second light radiation enhances the second adhesive strength; and/or
wherein the second film (2) is formed with a photopolymerization layer on the side towards the first film (1), which under irradiation with the second light radiation enhances the second adhesive strength; and/or
wherein the first film (1) and/or the second film (2) is/are adapted to attenuate light components with a wavelength shorter than 400 nm by at least 50 %; and/or
wherein the first film (1) and/or the second film (2) substantially consists/consist of one of the following materials: polymer film, silicone film, polyethylene film, PLGA film; and/or
wherein the predetermined thickness of the film set lies in a range of 0.01 - 1 mm or 1 - 3 mm; and/or wherein a first thickness of the first film (1) lies in a range of 0.01 - 0.1 mm or 0.1 - 1 mm; and/or wherein a second thickness of the second film (2) is at least as great, that the third film (3), upon placement onto the inner sections (2a), once the outer section (2b) has been peeled off, does not touch the first film; and/or
wherein the second thickness lies in the range of 0.01 - 0.1 mm or 0.1 - 0.8 mm; and/or
wherein the first film (1), with a second film (2) peeled off, has an upper surface opposite the skin, which creates an adhesive strength with the third film (3), which is smaller than one tenth of the first adhesive strength; and/or
wherein the adhesive (10) is formed as a first adhesive layer and is arranged with the first film (1) on the side towards the skin; and/or
wherein the adhesive (10) substantially consists of one of the following materials or a mixture thereof: component adhesive, organic and biological polymers, in which the polymerization is initiated chemically or thermally or photo-activated or by ionizing radiation;
wherein the predetermined depth from the skin surface down into the skin lies in a range of 50 - 500 µm or 0.5 - 1 mm or 1 - 3 mm; and/or
wherein the fourth adherence, with which the punched parts of full-thickness skin (12) are held in the donor skin, lies in a range of 0.08 - 4 N/cm²; and/or
wherein the hollow-cylinder inner diameter of the hollow cylindrical shape has an inner diameter of 0.1 - 0.3 mm or 0.3 - 0.5 mm or 0.1 - 1 mm; and/or
wherein the hollow-cylinder distance is defined as a smallest distance of two adjacent hollow cylindrical cuts and the hollow-cylinder distance lies in a range of 0.1 - 1 mm or 1 - 3 mm; and/or
wherein the first film (1) and/or the second film (2) and/or the third film (3) are biocompatible; and/or
wherein the first film (1) and/or the second film (2) and/or the third film (3) are adapted to be biodegradable.

14. The device set according to one of the preceding claims, wherein the first film (1) and/or the second film (2) are formed with a photopolymerization layer therebetween, which upon irradiation with the second light radiation enhances the second adhesive strength, wherein the cutting device is provided with a second light source for generating the second light radiation, wherein the second light radiation is generated such that only the inner sections (2a) of the second film (2) are irradiated.

15. The device set according to one of the preceding claims, wherein the third film (3) and the top side of the second film (2) are adapted to create the third adhesive strength under the influence of a first light radiation (hf) from a first light source (11); and/or wherein the third film (3) is adapted to harden under the influence of the first light radiation (hf) from the first light source (11); and/or
wherein the third film (3) has, at least on its bottom side oriented towards the top side of the second film (2), a photoactivatable polymer layer, which upon irradiation with the first light radiation (hf) forms a covalent and linking bond with the second film (2); and/or
wherein the second film (2) has, at least on its top side, a photoactivatable polymer layer, which upon irradiation with the first light radiation (hf) forms a covalent and linking bond with the third film (3); and/or
wherein the third film (3) substantially consist of one of the following materials or a mixture thereof: polymer film, organic polymers, biological polymers.

## Revendications

1. Ensemble de dispositifs pour générer une matrice de microgreffe avec une pluralité de greffons cutanés complets (12) découpés de la peau d'une zone de prélèvement, comprenant:
un ensemble de films ayant une épaisseur prédéterminée comportant au moins un premier film (1) et un second film (2), ledit premier film (1) étant conçu de façon à être fixé à la peau avec une face inférieure plate et à adhérer, avec une face supérieure opposée, à une face inférieure dudit second film (2) de manière coplanaire avec une seconde force adhésive et à être pelable l'un de l'autre;
un adhésif (10) pour fixer ladite face inférieure dudit premier film (1) à la peau, une première force adhésive étant créée entre ladite face inférieure dudit premier film (1) et la peau, force qui est supérieure à la seconde force adhésive, de sorte que ledit second film (2) soit pelable dudit premier film (1) fixé à la peau;
un dispositif de coupe ayant un adaptateur qui est conçu de façon à créer dans la zone d'enlèvement de la peau une distance prédéfinie à la peau sous-jacente tenant compte de l'épaisseur de l'ensemble de films lorsqu'il est pressé sur l'ensemble de films, ledit dispositif de coupe étant conçu de façon à produire une pluralité de découpes cylindriques creuses parallèles les unes aux autres avec un diamètre intérieur de cylindre creux et une distance de cylindre creux les unes des autres, chacune verticalement à l'ensemble de films et jusqu'à une profondeur prédéterminée dans la peau sous-jacente, de sorte que ladite pluralité des découpes cylindriques creuses soit produite sous la forme d'une matrice dans la zone de prélèvement de la peau, afin de diviser, par le biais de ces découpes cylindriques creuses, ledit ensemble de films en sections intérieures respectives avec une section intérieure respective (2a) dudit second film (2) laquelle se trouve au-dessus du greffon cutané complet découpé respectif (12), et une section extérieure respective avec une section extérieure (2b) dudit second film (2); et
un troisième film (3) d'une taille au moins aussi grande que la zone de prélèvement et qui est adapté pour créer une troisième force adhésive en contact avec une face supérieure dudit second film (2) opposé à la face inférieure dudit second film (2), les première, seconde et troisième forces adhésives étant supérieures à une quatrième adhérence qui maintient les greffons cutanés complets découpés (12) dans la peau.

2. Ensemble de dispositifs selon la revendication 1, dans lequel ledit dispositif de coupe comprend un élément de support (6) et une pluralité de micro-poinçons creux (4), qui sont respectivement formés le long d'un axe longitudinal et avec un bord de coupe à une extrémité inférieure et qui présentent un creux tubulaire pour produire les coupes cylindriques creuses correspondantes, lesdits micro-poinçons creux (4) étant reliés audit élément de support (6) avec une section supérieure opposée à l'arête de coupe, de telle sorte qu'ils forment, parallèlement les uns aux autres, un agencement des micro-poinçons creux (4) sous la forme d'une matrice, lesdits micro-poinçons creux (4) présentant une rigidité, un diamètre et une longueur tels qu'ils s'étendent à travers ledit ensemble de films jusqu'à la profondeur prédéterminée et puissent être pressés dans la peau,
lesdits micro-poinçons creux (4) présentant, dans une section avant d'une longueur de la profondeur prédéterminée et laquelle coupe dans la peau, une épaisseur de paroi de lame de 10 à 100 µm ou de 50 à 200 µm.

3. Ensemble de dispositifs selon la revendication 2, dans lequel ledit premier film (1) et/ou ledit second film (2) sont formés avec une couche de photopolymérisation entre eux, qui, lors de l'irradiation avec le second rayonnement lumineux, améliore ladite seconde force adhésive, ledit dispositif de coupe étant muni d'une seconde source de lumière pour générer ledit second rayonnement lumineux et ledit second rayonnement lumineux étant transféré de telle sorte qu'il passe à travers ledit creux interne dudit micro-poinçon creux respectif (4) vers ledit ensemble de films et ainsi seules lesdites sections intérieures (2a) dudit second film (2) sont irradiées.

4. Ensemble de dispositifs selon l'une des revendications précédentes 2 ou 3, dans lequel ledit dispositif de coupe comprend une pluralité de mandrins (4c), qui sont disposés dans lesdits micro-poinçons creux respectifs (4), ont un diamètre extérieur, qui est légèrement plus petit que le diamètre intérieur des dits micro-poinçons creux (4), afin de ne pas se coincer, et, par rapport aux dits micro-poinçons creux (4), peuvent être déplacés de manière contrôlable le long de l'axe longitudinal respectif, lesdits mandrins (4c), lors de l'enfoncement du dispositif de coupe dans la peau, libérant ledit creux respectif dans lesdits micro-poinçons creux (4), de sorte que ledit greffon cutané complet découpé respectif (12) puisse pénétrer dans ce creux, et lesdits mandrins (4c), lors du retrait des dits micro-poinçons creux (4) de la peau, maintenant les greffons cutanés complets découpés (12) sur la peau, lesdits micro-poinçons creux (4) étant retirés de la peau tandis que lesdits mandrins (4c) restent sur la peau.

5. Ensemble de dispositifs selon l'une des revendications précédentes 2 à 4, dans lequel ledit dispositif de coupe comprend un système de fluide fermé avec un récipient, un piston de déplacement contrôlable piloté par une commande, et un liquide, qui, dans une première étape, est déplacé dans lesdits micro-poinçons creux respectifs (4) par ledit piston de déplacement au moins jusqu'au bord de coupe respectif, la commande, dans une deuxième et troisième étape, entraînant ledit piston de déplacement de telle sorte que, pendant la deuxième et la troisième étape, le niveau du liquide dans lesdits micro-poinçons creux (4) par rapport à la peau est maintenu sensiblement constant, lorsque lesdits micro-poinçons creux (4) sont pressés jusqu'à la profondeur prédéterminée de la peau et retirés de celle-ci, afin de maintenir lesdits greffons cutanés complets découpés (12) dans la peau lors du retrait des micro-poinçons creux (4) de la peau, le liquide étant un liquide aqueux, qui comprend un des éléments suivants ou un mélange de ces éléments : eau, sel, épinéphrine, facteurs de croissance, vitamines, coenzymes, ou un autre médicament.

6. Ensemble de dispositifs selon l'une des revendications précédentes 2 à 5, dans lequel ledit dispositif de coupe est formé par lesdits micro-poinçons creux (4) étant reliés audit élément de support (6) par l'intermédiaire d'un premier actionneur respectif (5), ledit premier actionneur respectif (5) étant conçu et pilotable de façon à faire ledit micro-poinçon creux respectif (4) relié audit premier actionneur (5) vibrer dans le sens longitudinal et/ou dans un mouvement rotatif autour de l'axe longitudinal, une première fréquence de vibration et une première amplitude de vibration dans le sens longitudinal et/ou une deuxième fréquence de vibration et une deuxième amplitude de vibration pour le mouvement rotatif étant prédéterminées de telle sorte qu'une adhérence des dits greffons cutanés complets découpés (12) avec lesdits micro-poinçons creux (4) est largement évitée, lesdites première et deuxième fréquences de vibration se situant dans la plage de 20 kHz à 10 MHz et lesdites première et deuxième amplitudes de vibration se situant dans la plage de 1 µm à 300 µm; et/ou lesdits premiers actionneurs (5) étant des actionneurs piézoélectriques ou électromagnétiques ou d'autres actionneurs.

7. Ensemble de dispositifs selon l'une des revendications précédentes 2 à 6, dans lequel ledit dispositif de coupe est formé par le fait que ledit élément de support (6) est relié à un second support par l'intermédiaire d'au moins un second actionneur (8), et l'au moins un second actionneur (8) est conçu et pilotable de façon à déplacer ledit premier support (6) avec lesdits micro-poinçons creux (4) dans le sens longitudinal entre une première et une deuxième position, dans la première position, les micro-poinçons creux (4) étant encore disposés au-dessus dudit second film, sans le pénétrer, et, dans la deuxième position, pénétrant dans la profondeur prédéterminée sensiblement jusqu'à l'extrémité du derme (D1), lesdits micro-poinçons creux (4) étant insérés dans la peau par ledit second actionneur (8) avec une première vitesse et rétractés de celle-ci avec une deuxième vitesse; et/ou
lesdits seconds actionneurs (8) étant des actionneurs piézoélectriques ou électromagnétiques ou d'autres actionneurs.

8. Ensemble de dispositifs selon l'une des revendications précédentes 2 à 7, comprenant en outre un moyen de pelage (9) ou une languette en tant que section saillante ou en saillie d'une région de bord extérieur de ladite section extérieure (2b) dudit second film (2), ledit moyen de pelage (9) ou ladite languette étant un composant intégral dudit second film (2) ou d'une autre couche fixée sur celui-ci, afin de pouvoir maintenir ledit second film (2) sur celui-ci et le peler dudit premier film (1); ou
ledit moyen de pelage (9) étant un agencement aspirant ou adhésif lequel est disposé et formé entre lesdits micro-poinçons creux (4) au-dessus de ladite section extérieure (2b) dudit second film (2) pour attirer ou retirer ladite section extérieure (2b) en aspirant ou collant.

9. Ensemble de dispositifs selon l'une des revendications précédentes 2 à 8, dans lequel la pluralité des dits micro-poinçons creux (4) présentent respectivement un point de contact (4a) à l'extrémité respective opposée à l'arête de coupe, qui, par l'intermédiaire de points de contre-contact respectifs correspondants, sont reliés audit élément de support (6) et peuvent être séparés dudit élément de support (6) et peuvent être reliés audit élément de support (6).

10. Ensemble de dispositifs selon l'une des revendications précédentes 2 à 8, dans lequel la pluralité des dits micro-poinçons creux (4) sont découpés et inversés à partir d'une tôle métallique commune (4b), une extrémité supérieure de la tôle, opposée aux arêtes de coupe, étant plate et en forme de plaque et ayant des trous, qui correspondent aux dits creux tubulaires des dits micro-poinçons creux (4), ladite extrémité supérieure de la tôle ayant un point de contact avec un point de contre-contact correspondant à celui-ci sur ledit élément de support (6), ledit point de contact étant relié audit élément de support (6) et pouvant être séparé dudit élément de support (6).

11. Ensemble de dispositifs selon l'une des revendications précédentes 2 à 10, dans lequel lesdits micro-poinçons (4) sont essentiellement constitués du matériau suivant ou d'un mélange ou d'une combinaison de ceux-ci : titane, acier inoxydable, composite renforcé par des fibres, matériau biodégradable, acide poly(lactique-co-glycolique) (PLGA), saccharide, polymères, protéines, protéines de soie d'araignée, collagène/gélatine, réticulé et minéralisé selon le cas, cellulose; et/ou
lesdits micro-poinçons creux (4) ayant un revêtement monocouche conçu pour obtenir le moins d'adhérence possible avec la peau; et/ou
lesdits micro-poinçons creux (4) ayant un revêtement du type suivant ou une combinaison de ceux-ci :
parylène, ASD (atomic single layer déposition), un revêtement hydrophobe, un revêtement polarisé, ayant une surface chargée négativement, un revêtement SAM (SAM = self-assembling monolayer), un revêtement avec un composé fluoré ou du téflon; et/ou
ledit dispositif de coupe comprenant un liquide et une pompe reliée à celui-ci et qui est reliée aux dits creux des dits micro-poinçons creux (4) et déplace le liquide en quantité prédéterminée dans lesdits micro-poinçons creux (4) ou en retour de ceux-ci, ledit liquide comprenant un des éléments suivants ou un mélange de ceux-ci : eau, sel, épinéphrine, facteurs de croissance, vitamines, coenzymes, ou un autre médicament.

12. Ensemble de dispositifs selon la revendication 1, dans lequel ledit dispositif de coupe comprend un laser avec une optique, afin de générer un tel faisceau laser, qui produise ladite pluralité de coupes cylindriques creuses de la profondeur prédéterminée à travers ledit ensemble de films dans la peau; et/ou ledit laser ayant une longueur d'onde de lumière dans la plage de 700 à 10.000 nm et étant un type de laser IR et/ou un laser femtoseconde.

13. Ensemble de dispositifs selon l'une des revendications précédentes, dans lequel ledit premier film (1) est formé avec une couche de photopolymérisation sur le côté vers ledit second film (2), qui sous irradiation avec un second rayonnement lumineux améliore ladite seconde force adhésive; et/ou
ledit second film (2) étant formé avec une couche de photopolymérisation sur le côté vers ledit premier film (1), qui sous l'irradiation avec le second rayonnement lumineux améliore la seconde force adhésive; et/ou
ledit premier film (1) et/ou ledit second film (2) étant adapté(s) pour atténuer les composantes de la lumière d'une longueur d'onde plus courte que 400 nm d'au moins 50 %; et/ou
ledit premier film (1) et/ou ledit second film (2) étant essentiellement constitué(s) de l'un des matériaux suivants : film polymère, film de silicone, film de polyéthylène, film de PLGA; et/ou l'épaisseur prédéterminée dudit ensemble de films se situant dans une plage de 0,01 à 1 mm ou 1 à 3 mm; et/ou
une première épaisseur dudit premier film (1) se situant dans une plage de 0,01 à 0,1 mm ou 0,1 à 1 mm; et/ou
une seconde épaisseur dudit second film (2) étant au moins aussi grande, que le troisième film (3), lors de son placement sur lesdites sections intérieures (2a), ne touche pas ledit premier film une fois que ladite section extérieure (2b) a été décollée; et/ou ladite seconde épaisseur se situant dans la plage de 0,01 à 0,1 mm ou 0,1 à 0,8 mm; et/ou
ledit premier film (1), avec un second film (2) décollé, ayant une surface supérieure opposée à la peau, ce qui crée une force adhésive avec ledit troisième film (3), qui est inférieure à un dixième de ladite première force adhésive; et/ou
ledit adhésif (10) étant formé comme une première couche adhésive et disposé avec le premier film (1) sur le côté vers la peau; et/ou
ledit adhésif (10) étant essentiellement constitué de l'un des matériaux suivants ou d'un mélange de ceux-ci: adhésif composant, polymères organiques et biologiques, dans lequel la polymérisation est initiée chimiquement ou thermiquement ou photo-activée ou par rayonnement ionisant;
ladite profondeur prédéterminée depuis la surface de la peau jusque dans la peau se situant dans une plage de 50 à 500 µm ou 0,5 à 1 mm ou 1 à 3 mm; et/ou la quatrième adhérence, maintenant les greffons cutanés complets découpés (12) dans la peau du donneur, se situant dans une plage de 0,08 à 4 N/cm2; et/ou
le diamètre intérieur du cylindre creux de la forme cylindrique creuse ayant un diamètre intérieur de 0,1 à 0,3 mm ou de 0,3 à 0,5 mm ou de 0,1 à 1 mm; et/ou la distance entre cylindres creux étant définie comme la plus petite distance de deux coupes cylindriques creuses adjacentes et la distance entre cylindres creux se situant dans une plage de 0,1 à 1 mm ou de 1 à 3 mm; et/ou
ledit premier film (1) et/ou ledit second film (2) et/ou ledit troisième film (3) étant biocompatibles; et/ou
ledit premier film (1) et/ou ledit second film (2) et/ou ledit troisième film (3) étant conçus pour être biodégradables.

14. Ensemble de dispositifs selon l'une des revendications précédentes, dans lequel ledit premier film (1) et/ou ledit second film (2) sont formés avec une couche de photopolymérisation entre eux, qui, lors de l'irradiation avec le second rayonnement lumineux, améliore ladite seconde force adhésive, ledit dispositif de coupe étant muni d'une seconde source de lumière pour générer ledit second rayonnement lumineux, ledit second rayonnement lumineux étant généré de telle sorte que seules lesdites sections internes (2a) dudit second film (2) sont irradiées.

15. Ensemble de dispositifs selon l'une des revendications précédentes, dans lequel ledit troisième film (3) et ladite face supérieure dudit second film (2) sont adaptés pour créer ladite troisième force adhésive sous l'influence d'un premier rayonnement lumineux (hf) provenant d'une première source lumineuse (11); et/ou
ledit troisième film (3) étant conçu pour durcir sous l'influence dudit premier rayonnement lumineux (hf) provenant de ladite première source lumineuse (11); et/ou
ledit troisième film (3) possédant, au moins sur sa face inférieure orientée vers la face supérieure dudit second film (2), une couche de polymère photoactivable, qui, lors de l'irradiation avec ledit premier rayonnement lumineux (hf), forme une liaison covalente et de liaison avec ledit second film (2); et/ou
ledit second film (2) ayant, au moins sur sa face supérieure, une couche de polymère photoactivable, qui, lors de l'irradiation avec ledit premier rayonnement lumineux (hf), forme une liaison covalente et de liaison avec ledit troisième film (3); et/ou ledit troisième film (3) étant essentiellement constitué de l'un des matériaux suivants ou d'un mélange de ceux-ci : film polymère, polymères organiques, polymères biologiques.
